(19) Europäisches Patentamt
European Patent Office
Office européen des brevets

(11) **EP 3 383 466 B1**

(12) **EUROPÄISCHE PATENTSCHRIFT**

(45) Veröffentlichungstag und Bekanntmachung des Hinweises auf die Patenterteilung:
**12.02.2020   Patentblatt 2020/07**

(21) Anmeldenummer: **16798412.9**

(22) Anmeldetag: **21.11.2016**

(51) Int Cl.:
**A61M 16/01** (2006.01)

(86) Internationale Anmeldenummer:
**PCT/EP2016/001951**

(87) Internationale Veröffentlichungsnummer:
**WO 2017/092850 (08.06.2017 Gazette 2017/23)**

(54) **ANÄSTHESIEBEATMUNGSVORRICHTUNG ZUR AUTOMATISIERTEN BEATMUNG SOWIE ZUR DETEKTION EINES BETRIEBSZUSTANDES HINSICHTLICH DER AUTOMATISIERTEN BEATMUNG**

ANESTHESIA VENTILATION DEVICE FOR PROVIDING AUTOMATED VENTILATION AND FOR DETECTING AN OPERATING STATE WITH REGARD TO THE AUTOMATED VENTILATION

DISPOSITIF DE VENTILATION POUR ANESTHÉSIE, PERMETTANT UNE VENTILATION AUTOMATISÉE ET LA DÉTECTION D'UN ÉTAT DE FONCTIONNEMENT DE LA RESPIRATION AUTOMATISÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2015   DE 102015015441**

(43) Veröffentlichungstag der Anmeldung:
**10.10.2018   Patentblatt 2018/41**

(73) Patentinhaber: **Drägerwerk AG & Co. KGaA**
**23558 Lübeck (DE)**

(72) Erfinder:
• **BUSCHKE, Wilfried**
  **23560 Lübeck (DE)**
• **HÖRMANN, Christoph**
  **3240 Mank (AT)**
• **MERSMANN, Stefan**
  **23564 Lübeck (DE)**

(56) Entgegenhaltungen:
EP-A1- 1 974 763      WO-A1-2009/050736
DE-A1-102013 002 408      DE-B3-102005 012 340

**Beschreibung**

Aus dem Stand der Technik sind Anästhesiebeatmungsvorrichtungen sowie Verfahren bekannt, bei welchen eine druckgesteuerte Beatmung eines Patienten vorgenommen wird.

Ferner ist es bekannt, im Rahmen solcher Beatmungsverfahren ein sogenanntes Weaning durchzuführen, bei welchem der Patient in einer sogenannten Komfortzone gehalten wird, wobei für den Zweck des Weaning im Rahmen einer druckunterstützenden Beatmung ein Solldruckwert bzw. ein Druckunterstützungswert in Abhängigkeit eines detektierten Tidalvolumens und einer endexspiratorischen Kohlendioxidkonzentration angepasst wird. Derartige Verfahren sind auch als sogenannte "Smart-Care/PS" Verfahren bekannt.

Aufgabe der vorliegenden Erfindung ist es, mittels einer Anästhesiebeatmungsvorrichtung eine druckgesteuerte Beatmung eines Patienten durchzuführen, bei welcher die Anästhesiebeatmungsvorrichtung nicht nur eine automatisierte Beatmung des Patienten durchführt, sondern bei welcher auch ein Wechsel zwischen Betriebsmodi in vorteilhafter Weise möglich ist. DE102013002408 beschreibt eine Vorrichtung zur Atemunterstützung.

Die erfindungsgemäße Aufgabe wird gelöst durch eine Anästhesiebeatmungsvorrichtung nach dem Anspruch 1. Ferner wird die erfindungsgemäße Aufgabe gelöst durch eine Recheneinheit nach dem Patentanspruch 10.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorgeschlagen wird eine Anästhesiebeatmungsvorrichtung zur automatisierten Beatmung eines Patienten, aufweisend einen exspiratorischen Port und einen inspiratorischen Port zum Anschluss eines dem Patienten zugewandten Beatmungsschlauches für ein Atemgas, eine Atemgasfördereinheit, wenigstens einen Volumenstromsensor zum Erfassen eines Volumenstroms des Atemgases, wenigstens einen Atemgassensor zum Erfassen einer Kohlendioxidkonzentration sowie wenigstens einen Drucksensor zum Erfassen eines Drucks des Atemgases und ferner wenigstens eine Recheneinheit, welche dazu ausgebildet ist, in einem ersten Betriebsmodus die Atemgasfördereinheit in Abhängigkeit einer vorgegebenen Beatmungsfrequenz, des erfassten Drucks und eines vorgegebenen Solldruckwertes anzusteuern, wobei die Recheneinheit ferner dazu ausgebildet ist, auf Basis des erfassten Volumenstroms und der erfassten Kohlendioxidkonzentration ein Vorliegen eines erwünschten Betriebszustand hinsichtlich der automatisierten Beatmung zu detektieren und bei Detektion des Betriebszustandes einen Wechsel in einen zweiten Betriebsmodus zu ermöglichen, in welchem die Recheneinheit die Atemgasfördereinheit derart ansteuert, dass eine druckgesteuerte oder eine druckunterstützende Beatmung durchgeführt wird.

Die erfindungsgemäße Vorrichtung ist vorteilhaft, da diese Vorrichtung anhand des erfassten Volumenstroms sowie einer erfassten Kohlendioxidkonzentration detektieren kann, dass ein gewünschter Betriebszustand der Beatmung vorliegt. Ein solcher Betriebszustand ist dann gegeben, wenn eine aus dem Volumenstrom abgeleitete Größe, vorzugsweise ein Tidalvolumen, und eine aus der Kohlendioxidkonzentration, vorzugsweise eine endexspiratorische Kohlendioxidkonzentration, jeweilige gewünschte Korrelationen bzw. Wertekonstellationen zu jeweiligen vorgegebenen Grenzwerten aufweisen. Hierdurch kann ggf. abgeleitet werden, dass ein Wechsel in einen zweiten Betriebsmodus der Beatmung für den Patienten hinnehmbar ist.

Vorzugsweise ist die Recheneinheit dazu ausgebildet, den Wechsel in den zweiten Betriebsmodus dann zu ermöglichen, wenn der Betriebszustand innerhalb eines vorgegebenen Zeitraumes detektiert wird. Hierdurch wird sichergestellt, dass nicht immer weiter fortlaufend versucht bzw. abgewartet wird, dass sich der Betriebszustand einstellt, sondern das Verfahren leitet nur dann in den zweiten Betriebsmodus über, wenn innerhalb eines vernünftigen bzw. vorgegebenen Zeitraumes der Betriebszustand erreicht wird. Vorzugsweise nimmt hierbei die Recheneinheit den Wechsel in den zweiten Betriebsmodus automatisch vor. Vorzugsweise wird auch bei diesem automatischen Wechseln ein Ausgabesignal ausgegeben, welches das Vorliegen des erwünschten Betriebszustandes bzw. den Wechsel in den zweiten Betriebsmodus indiziert. Hierdurch wird ein Kliniker informiert, dass der Betriebszustand erreicht ist, und dass ein Betriebsmoduswechsel erfolgt.

Vorzugsweise ist die Recheneinheit dazu ausgebildet, bei Detektion des Betriebszustandes ein Ausgabesignal auszugeben, welches das Vorliegen des erwünschten Betriebszustandes indiziert, sowie in Abhängigkeit eines Eingabesignals in den zweiten Betriebsmodus zu wechseln. Liegt der Betriebszustand vor, so kann dies ein Hinweis für einen Kliniker sein, dass der Patient möglicherweise ein gewünschtes Atemverhalten aufweist. Die Ausgabe des Ausgabesignals, welches das Vorliegen des Betriebszustandes der Vorrichtung hinsichtlich der automatisierten Beatmung indiziert, kann dann für einen Kliniker ein Hinweis sein, ein Überleiten von dem ersten Betriebsmodus hin zu einem zweiten Betriebsmodus in Betracht zu ziehen. Der Kliniker kann dann mittels eines Eingabesignals einen Übergang in den zweiten Betriebsmodus herbeiführen. In dem zweiten Betriebsmodus kann dann entweder eine druckgesteuerte Beatmung oder eine druckunterstützende Beatmung durchgeführt werden. Im Rahmen einer Anästhesie ist es eine übliche Situation, dass ein Patient mittels druckgesteuerter Beatmung beatmet wird. Für einen Kliniker ist es mitunter eine Aufgabe, dass er einen Zeitpunkt bestimmen muss, zu welchem er einen Patient durch die Beatmungsvorrichtung in einem ganz bestimmten Betriebsmodus, vorzugsweise den zweiten Betriebsmodus, beatmen lassen möchte. Da der Kliniker hierbei darauf abstellen muss, ob der Patient stabil genug ist, um mittels eines solchen zweiten Betriebsmodus beatmet werden zu können, muss der Kliniker hierfür möglicherweise auf Eigenschaften einer Atemaktivität des Patienten abstellen. Hierfür kann der Hinweis auf Vorliegen des erwünschten Betriebszustandes eine Hilfestellung für den Kliniker sein. Die erfindungsgemäße Vorrichtung automatisiert die Beatmung vorzugsweise in besonders vorteilhafter Weise, da die Vorrichtung auf Basis des Volumenstroms sowie der Kohlendioxidkonzentration das Vorliegen des Betriebszustandes hinsichtlich der automatisierten Beatmung detektieren kann und dieses dann dem Kliniker durch Ausgabe des Ausgabesignals anzeigt. Entscheidet der Kliniker sich von sich aus dafür, dass er tatsächlich die Vorrichtung in den weiteren zweiten Betriebsmodus überführen möchte, so kann er dies durch eine Eingabe bzw. ein Eingabesignal automatisiert unter seiner Kontrolle herbeiführen. Es bleibt somit in der Hand des Klinikers, ob der zweite Betriebsmodus durchgeführt wird, wobei aber der Kliniker in seiner Entscheidung durch Ausgabe des Ausgabesignals unterstützt wird.

Gemäß einer vorteilhaften Ausgestaltung wird vorgeschlagen, dass die Recheneinheit dazu ausgebildet ist, in dem ersten Betriebsmodus auf Basis des erfassten Volumenstroms ein dem Patienten zugeführtes Tidalvolumen zu bestimmen, und ferner auf Basis der erfassten Kohlendioxidkonzentration eine endexspiratorische Kohlendioxidkonzentration zu bestimmen sowie ferner eine Anpassung des Solldruckwertes und eine Anpassung der Beatmungsfrequenz in Abhängigkeit des bestimmten Tidalvolumens, eines oberen Volumengrenzwertes, eines unteren Volumengrenzwertes, der bestimmten endexspiratorischen Kohlendioxidkonzentration, eines oberen Konzentrationsgrenzwertes und eines unteren Konzentrationsgrenzwertes vorzunehmen. Diese Ausgestaltung der Vorrichtung ist vorteilhaft, da auf Basis der definierten Grenzwerte sowie des gemessenen Tidalvolumens als auch der endexspiratorischen Kohlendioxidkonzentration im Zuge des ersten Betriebsmodus, bei welchem eine druckgesteuerten Beatmung durchgeführt wird, sowohl der Solldruckwert als auch die Beatmungsfrequenz angepasst werden können, um das durch den Patienten geatmete Tidalvolumen als auch die endexspiratorische Kohlendioxidkonzentration innerhalb durch die Grenzwerte definierter Grenzen, also einer Komfortzone, halten zu können. Vorzugsweise detektiert die Anästhesiebeatmungsvorrichtung das Vorliegen des erwünschten Betriebszustandes in dem Fall, dass das Tidalvolumen zwischen dem oberen und dem unteren Volumengrenzwertliegt und dass ferner die endexspiratorische Kohlendioxidkonzentration zwischen dem oberen Konzentrationsgrenzwert und dem unteren Konzentrationsgrenzwert liegt.

Vorzugsweise weist die Anästhesiebeatmungsvorrichtung ferner wenigstens einen Atemgassensor zum Erfassen einer Anästhesiegaskonzentration des Atemgases auf, wobei der erwünschte Betriebszustand ein erster erwünschter Betriebszustand ist, und wobei die Recheneinheit ferner dazu ausgebildet ist, in dem zweiten Betriebsmodus auf Basis der erfassten Anästhesiegaskonzentration ein Vorliegen eines zweiten erwünschten Betriebszustand hinsichtlich der automatisierten Beatmung zu detektieren und bei Detektion des zweiten Betriebszustandes ein zweites Ausgabesignal auszugeben, welches das Vorliegen des zweiten erwünschten Betriebszustandes indiziert. Diese Ausgestaltung der Erfindung ist vorteilhaft, da es somit möglich ist, während des zweiten Betriebsmodus den Kliniker darüber zu informieren, dass ein weiterer, zweiter erwünschter Betriebszustand hinsichtlich der automatisierten Beatmung erreicht ist. Der Kliniker kann dann möglicherweise eine Veränderung der automatisierten Beatmung in Betracht ziehen. Vorzugsweise erfolgt bei Detektion des zweiten Betriebszustandes ferner ein Übergang von dem zweiten Betriebsmodus in einen dritten Betriebsmodus, in welchem die Recheneinheit die Atemgasfördereinheit derart ansteuert, dass eine druckunterstützende Beatmung durchgeführt wird, automatisch.

Vorzugsweise ist die Recheneinheit ferner dazu ausgebildet ist, in dem zweiten Betriebsmodus das Vorliegen des zweiten erwünschten Betriebszustand hinsichtlich der automatisierten Beatmung ferner in Abhängigkeit eines Informationssignals, welches einen Betriebszustand eines Vapors indiziert, zu detektieren. Diese Ausgestaltung der Erfindung ist vorteilhaft, da hiermit im Zuge der Überprüfung des Vorliegens des zweiten Betriebszustands auch darauf abgestellt werden kann, ob der Vapor als Hauptquelle des Anästhesiemittels einen erwünschten Betriebszustand aufweist. Ist beispielsweise der Vapor in dem erwünschten Betriebszustand ausgestaltet, so kann das Ausgabesignal durch den Kliniker als Hinweis gewertet werden, dass eine Endphase der Anästhesiebeatmung erreicht sein könnte.

Vorzugsweise ist die Recheneinheit dazu ausgebildet, bei Detektion des zweiten Betriebszustandes in Abhängigkeit eines weiteren Eingabesignals in einen dritten Betriebsmodus zu wechseln, in welchem die Recheneinheit die Atemgasfördereinheit derart ansteuert, dass eine druckunterstützende Beatmung durchgeführt wird. Diese Ausgestaltung der Erfindung ist vorteilhaft, da das Ausgabesignal als Hinweis auf das Vorliegen des zweiten Betriebszustandes durch den Kliniker als Hinweis gewertet werden kann, um einen Wechsel in einen dritten Betriebsmodus in Betracht zu ziehen, in welchem der Patient lediglich eine druckunterstützende Beatmung erfährt. Eine druckunterstützende Beatmung stellt darauf ab, dass der Patient selber Spontanatemaktivität zeigt, welches gerade zum Ende der Anästhesiebeatmung nach Unterbinden der Zuführung weiteren Anästhetikums erwünscht ist. Der Kliniker muss hierbei nicht selber auf den Modus der druckunterstützenden Beatmung durch Einstellung bestimmter Parameter wechseln, sondern die Recheneinheit wechselt in einen solchen Modus der druckunterstützenden Beatmung aufgrund der Eingabe des Klinikers in automatisierter Weise unter Kontrolle des Klinikers.

Vorzugsweise ist die Recheneinheit dazu ausgebildet, in dem zweiten Betriebsmodus die Atemgasfördereinheit in Abhängigkeit des erfassten Drucks und eines zweiten vorgegebenen Solldruckwertes für eine druckgesteuerte oder eine druckunterstützende Beatmung anzusteuern, ferner auf Basis des erfassten Volumenstroms ein dem Patienten zugeführtes Tidalvolumen zu bestimmen, auf Basis der erfassten Kohlendioxidkonzentration eine endexspiratorische Kohlendioxidkonzentration zu bestimmen sowie ferner eine Anpassung des Solldruckwertes und eine Anpassung einer Beatmungsfrequenz in Abhängigkeit des bestimmten Tidalvolumens, eines oberen Volumengrenzwertes, eines unteren Volumengrenzwertes, der bestimmten endexspiratorischen Kohlendioxidkonzentration, eines oberen Konzentrationsgrenzwertes und eines unteren Konzentrationsgrenzwertes vorzunehmen. Diese Ausgestaltung der Erfindung ist vorteilhaft, da während des zweiten Betriebsmodus die druckgesteuerte oder druckunterstützende Beatmung nicht nur hinsichtlich des Solldruckwertes, sondern auch einer Beatmungsfrequenz in Abhängigkeit des Tidalvolumens und auch der endexspiratorischen Kohlendioxidkonzentration angepasst wird.

Vorzugsweise ist die Recheneinheit dazu ausgebildet, in dem dritten Betriebsmodus die Atemgasfördereinheit in Abhängigkeit des erfassten Drucks und eines vorgegebenen Solldruckwertes anzusteuern, wobei die Recheneinheit ferner dazu ausgebildet ist, in dem dritten Betriebsmodus eine Anpassung des Solldruckwertes in Abhängigkeit der erfassten Anästhesiegaskonzentration vorzunehmen. Diese Ausgestaltung der Erfindung ist vorteilhaft, da die Vorrichtung somit in dem dritten Betriebsmodus die Anästhesiegaskonzentration berücksichtigen kann, um in dieser Phase der druckunterstützenden Beatmung den Solldruckwert anzupassen.

Ferner wird ein nicht von der Erfindung umfasstes Verfahren zum Betreiben einer Anästhesiebeatmungsvorrichtung für eine automatisierte Beatmung eines Patienten vorgestellt, aufweisend: Zuführen eines Atemgases zu einem Patienten über einen inspiratorischen Port und Rückführen des Atemgases über einen exspiratorischen Port durch Betreiben einer Atemgasfördereinheit, Erfassen eines Volumenstroms des Atemgases mittels wenigstens eines Volumenstromsensors, Erfassen einer Kohlendioxidkonzentration des Atemgases mittels wenigstens eines Atemgassensors, Erfassen eines Drucks des Atemgases mittels wenigstens eines Drucksensors, sowie, in einem ersten Betriebsmodus, Ansteuern der Atemgasfördereinheit in Abhängigkeit einer vorgegebenen Beatmungsfrequenz, des erfassten Drucks und eines vorgegebenen Solldruckwertes mittels wenigstens einer Recheneinheit, gekennzeichnet durch Detektieren eines Vorliegens eines erwünschten Betriebszustand hinsichtlich der automatisierten Beatmung auf Basis des erfassten Volumenstroms und der erfassten Kohlendioxidkonzentration und, bei Detektion des Betriebszustandes, Ermöglichen eines Wechsels in einen zweiten Betriebsmodus, in welchem die Recheneinheit die Atemgasfördereinheit derart ansteuert, dass eine druckgesteuerte oder eine druckunterstützende Beatmung durchgeführt wird.

Vorzugsweise ist das nicht von der Erfindung umfasste Verfahren dadurch gekennzeichnet, dass bei Detektion des Betriebszustandes ein Ausgeben eines Ausgabesignals, welches das Vorliegen des erwünschten Betriebszustandes indiziert, sowie, dass in Abhängigkeit eines Eingabesignals ein Wechseln in einen zweiten Betriebsmodus erfolgt, in welchem die Atemgasfördereinheit mittels der Recheneinheit derart angesteuert wird, dass eine druckgesteuerte oder eine druckunterstützende Beatmung durchgeführt wird.

Ferner wird eine Recheneinheit für eine Anästhesiebeatmungsvorrichtung zur automatisierten Beatmung eines Patienten vorgeschlagen, welche ausgebildet ist zum Erfassen eines Volumenstromsignals, welches einen Volumenstrom eines Atemgases indiziert, ferner zum Erfassen eines Kohlendioxidkonzentrationssignals, welches eine Kohlendioxidkonzentration des Atemgases indiziert, ferner zum Erfassen eines Drucksignals, welches einen Druck des Atemgases indiziert, in einem ersten Betriebsmodus. Die Recheneinheit ist ferner ausgebildet zum Bereitstellen eines Ansteuersignals für eine Atemgasfördereinheit, wobei die Recheneinheit das Ansteuersignals in Abhängigkeit einer vorgegebenen Beatmungsfrequenz, des erfassten Drucksignals und eines vorgegebenen Solldruckwertes, wobei die Recheneinheit ferner ausgebildet ist, auf Basis des erfassten Volumenstroms und der erfassten Kohlendioxidkonzentration ein Vorliegen eines erwünschten Betriebszustand hinsichtlich der automatisierten Beatmung zu detektieren und, bei Detektion des Betriebszustandes, einen Wechsel in einen zweiten Betriebsmodus zu ermöglichen, in welchem die Recheneinheit die Atemgasfördereinheit derart ansteuert, dass eine druckgesteuerte oder eine druckunterstützende Beatmung durchgeführt wird.

Vorzugsweise ist die Recheneinheit dazu ausgebildet, bei Detektion des Betriebszustandes ein Ausgabesignal auszugeben, welches das Vorliegen des erwünschten Betriebszustandes indiziert, sowie in Abhängigkeit eines Eingabesignals in einen zweiten Betriebsmodus zu wechseln, in welchem Recheneinheit die Atemgasfördereinheit derart ansteuert, dass eine druckgesteuerte oder eine druckunterstützende Beatmung durchgeführt wird.

Ferner wird ein nicht von der Erfindung umfasstes Verfahren zum Betreiben einer Anästhesiebeatmungsvorrichtung zur automatisierten Beatmung eines Patienten vorgestellt, aufweisend: Erfassen eines Volumenstromsignals, welches einen Volumenstrom eines Atemgases indiziert, Erfassen eines Kohlendioxidkonzentrationssignals, welches eine Kohlendioxidkonzentration des Atemgases indiziert, Erfassen eines Drucksignals, welches einen Druck des Atemgases indiziert, in einem ersten Betriebsmodus, Bereitstellen eines Ansteuersignals für eine Atemgasfördereinheit, wobei die Recheneinheit das Ansteuersignal in Abhängigkeit einer vorgegebenen Beatmungsfrequenz, des erfassten Drucksignals und eines vorgegebenen Solldruckwertes bestimmt, gekennzeichnet durch Detektieren eines Vorliegens eines erwünschten Betriebszustand hinsichtlich der automatisierten Beatmung auf Basis des erfassten Volumenstroms und der erfassten Kohlendioxidkonzentration und, bei Detektion des Vorliegens, Ermöglichen eines Wechsels in einen zweiten Betriebsmodus, in welchem die Recheneinheit die Atemgasfördereinheit derart ansteuert, dass eine druckgesteuerte oder eine druckunterstützende Beatmung durchgeführt wird.

Vorzugsweise erfolgt bei Detektion des Vorliegens eine Ausgabe eines Ausgabesignals, welches das Vorliegen des erwünschten Betriebszustandes indiziert, sowie, in Abhängigkeit eines Eingabesignals, ein Wechsel in einen zweiten Betriebsmodus, in welchem die Atemgasfördereinheit derart angesteuert wird, dass eine druckgesteuerte oder eine druckunterstützende Beatmung durchgeführt wird.

Ferner wird vorgeschlagen, das zuvor beschriebene nicht von der Erfindung umfasste Verfahren zum Betreiben einer Anästhesiebeatmungsvorrichtung derart vorzusehen, dass das Verfahren mit Computerprogrammmitteln auf wenigstens einer Recheneinheit ausgeführt wird.

Im Folgenden wird die Erfindung anhand spezieller Ausführungsformen ohne Beschränkung des allgemeinen Erfindungsgedankens anhand der Figuren näher erläutert. Dabei zeigen:

Fig. 1   einen zeitlichen Druckverlauf sowie einen zeitlichen Volumenstromverlauf im Zuge einer Inspiration und einer Exspiration,

Fig. 2   einen zeitlichen Druckverlauf sowie einen zeitlichen Volumenstromverlauf im Rahmen einer druckgesteuerten Beatmung, bei welcher keine Spontanatemversuche eines Patienten ermöglicht werden sollen,

Fig. 3   einen zeitlichen Druckverlauf sowie ein zeitlichen Volumenstromverlauf im Rahmen einer druckunterstützenden Beatmung, welche im Rahmen von Spontanatemversuchen eines Patienten erfolgt,

Fig. 4   einen zeitlichen Druckverlauf sowie ein zeitlichen Volumenstromverlauf im Rahmen einer druckgesteuerten Beatmung, bei welcher Spontanatemversuche eines Patienten zugelassen werden,

Fig. 5   eine erfindungsgemäße Anästhesiebeatmungsvorrichtung,

Fig. 6   Schritte des nicht von der Erfindung umfassten Verfahrens in einem ersten Betriebsmodus und einem zweiten Betriebsmodus,

Fig. 7   vorgebbare Grenzwerte,

Fig. 8   Schritte, welche im Zuge eines zweiten Betriebsmodus durchgeführt werden sowie einen Übergang zu einem Betriebsmodus,

Fig. 9   einen zeitlichen Verlauf einer mittleren alveolären Anästhesiegaskonzentration,

Fig. 10   eine Tabelle mit durch einen Kliniker vorgebbaren Vorgabewerten,

Fig. 11   eine Tabelle mit möglichen Grenzwerten zur Durchführung des nicht von der Erfindung umfassten Verfahrens,

Fig. 12   jeweilige Tabellen zur Bestimmung von Ventilationsgraden bzw. Gasaustauschraten bezogen auf ein Tidalvolumen bzw. eine endexspiratorische Kohlendioxidkonzentration, und

Fig. 13   eine Übersicht verschiedener möglicher Betriebsmodi.

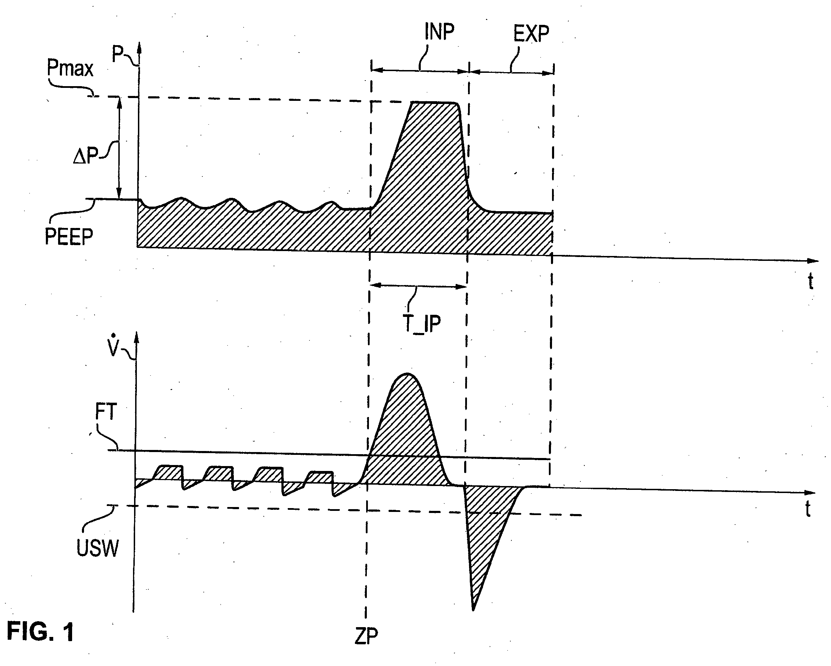

Figuren 1 bis 4 zeigen aus dem Stand der Technik bekannte Kurvenverläufe, anhand derer nun die Prinzipien einer Triggersteuerung, einer druckunterstützenden Beatmung, einer druckgesteuerten Beatmung ohne Zulassen von Spontanatemversuchen sowie, einer druckgesteuerten Beatmung mit Zulassen von Spontanatemversuchen erläutert werden.

Die Figur 1 zeigt einen Druckverlauf P sowie Volumenstromverlauf V über der Zeit t. Eine Flow-Triggerung besteht darin, dass ein Flow-Schwellenwert FT durch den Volumenstrom V zu einem Triggerzeitpunkt ZP überschritten wird, sodass dann der Druck von dem Mindestdruck PEEP (Positive End Exspiratory Pressure) um einen Erhöhungsdruck $\Delta P$ auf den Maximaldruck Pmax während der inspiratorische Phase INP gesteuert wird. Die exspiratorische Phase EXP folgt auf die inspiratorische Phase INP. Vorzugsweise kann der Beginn der exspiratorischen Phase EXP mittels eines unteren Schwellenwertes USW festgestellt werden, welcher mit einem negativen Signalverlauf durch den Volumenstrom V durchschritten werden muss.

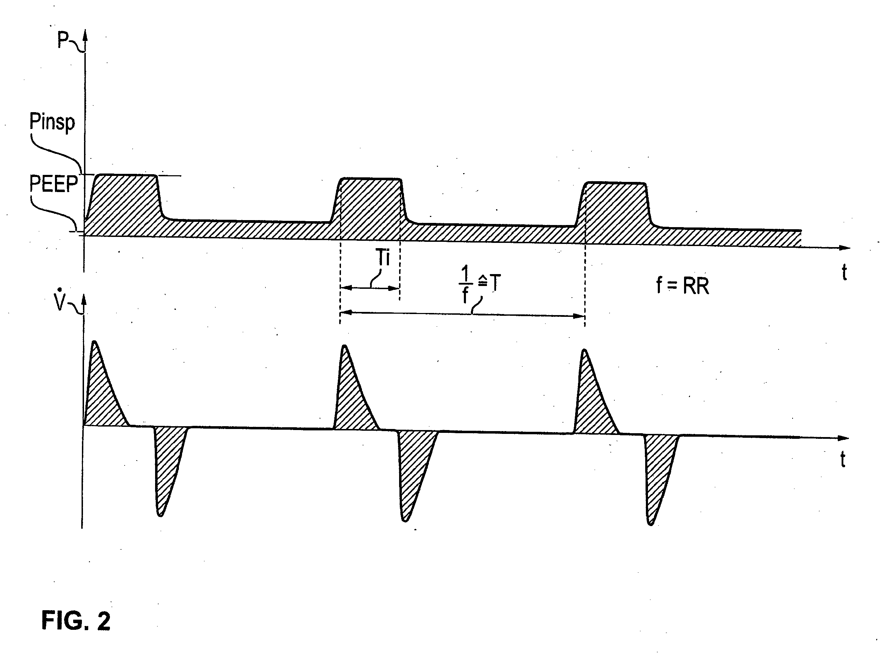

Die Figur 2 zeigt einen zeitlichen Druckverlauf P sowie einen Volumenstromverlauf V im Zuge einer reinen druckgesteuerten Beatmung, bei welcher der Druck in inspiratorischen Phasen auf einen Maximaldruckwert Pinsp gesteuert wird und bei welcher die inspiratorische Phase durch einen Zeitraum Ti vorgegeben wird. Die Frequenz f bzw. RR, mit welcher der Patient beatmet wird, wird hierbei fest vorgegeben. Es ergibt sich also ein Zeitraum T, welcher den Zeitraum zwischen den jeweiligen Anfängen jeweiliger aufeinanderfolgender inspiratorischer Phasen bestimmt.

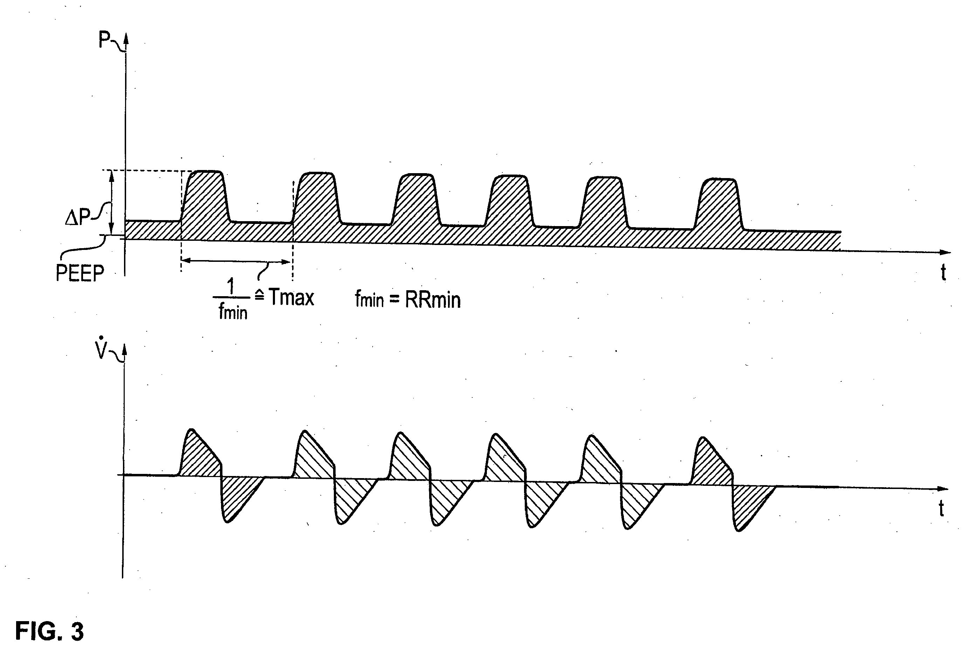

Die Figur 3 zeigt einen Druckverlauf P sowie einen Volumenstromverlauf V im Rahmen einer rein druckunterstützenden Beatmung. Unterschreitet die Atemfrequenz des Patienten eine gewisse Mindestfrequenz fmin bzw. RRmin, so kann vorzugsweise eine Warnung in Form eines Warnsignals ausgegeben werden. Der maximale Zeitraum Tmax zwischen den jeweiligen Anfängen jeweiliger inspiratorischer Phasen ist somit durch die Mindestatemfrequenz fmin bzw. RRmin vorgegeben. In einer inspiratorischen Phase wird der Druck P ausgehend von dem Mindestdruck PEEP um einen Druckhub bzw. Solldruckwert ΔP erhöht.

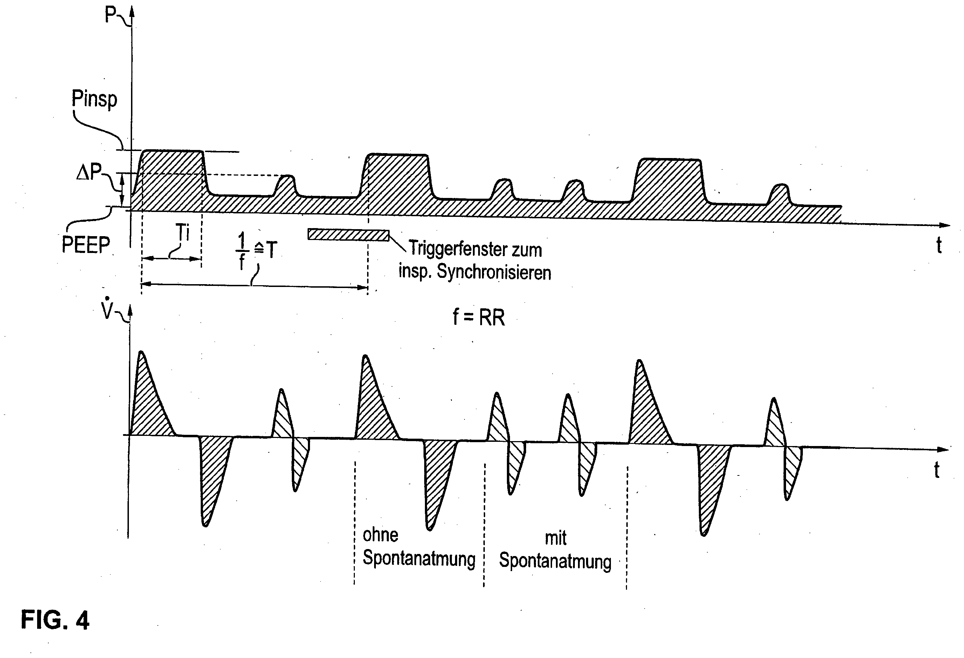

Die Figur 4 zeigt das Prinzip einer druckgesteuerten Beatmung, bei welcher aufgrund von Spontanatmung des Patienten auch jeweilige inspiratorische Phasen mit jeweiliger Druckunterstützung durchgeführt werden können.

Die Prinzipien der druckgesteuerten bzw. druckunterstützenden Beatmung sind auch aus der deutschen Patentanmeldung "Beatmungsvorrichtung und Verfahren zur automatisierten Beatmung eines Patienten", Anmelderin Drägerwerk AG & Co. KGaA, Erfinder Stefan Mersmann, Wilfried Buschke, Prof. Christoph Hörmann, eingereicht beim Deutschen Patent- und Markenamt am 02. Dezember 2015, entnehmbar. Ferner sind diese Prinzipien auch in den Dokumenten

- "Beatmungsmodi in der Intensivmedizin", Karin Deden, Dräger Medical GmbH
- "Gebrauchsanweisung"Zeus Infinity Empowered", Dräger Medical AG & Co. KG, 1. Ausgabe, Februar 2009

näher erläutert.

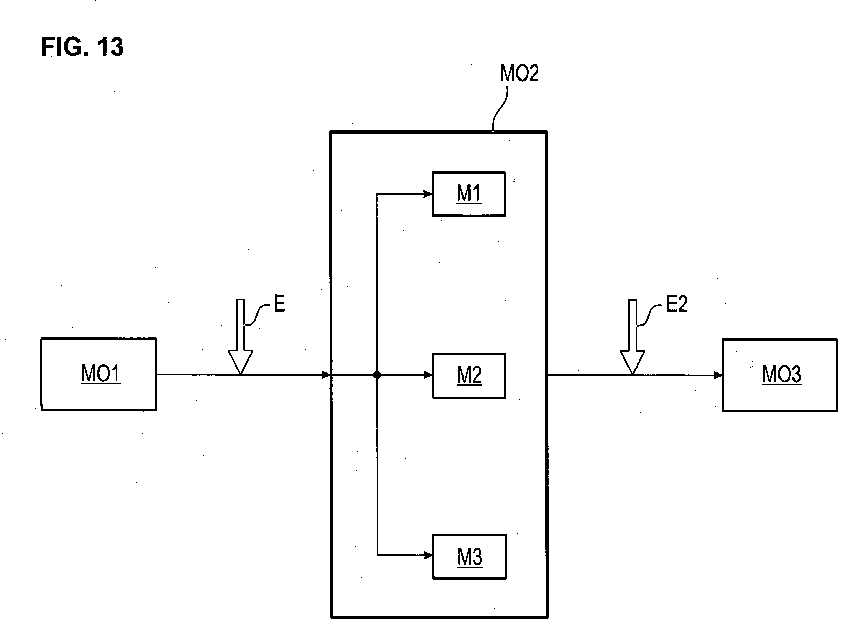

Die Figur 13 zeigt eine Übersicht über verschiedene mögliche Betriebsmodi, in welchen die vorgeschlagene Anästhesiebeatmungsvorrichtung vorzugsweise betrieben werden kann. In einem ersten Betriebsmodus MO1 wird eine rine druckgesteuerte Beatmung ohne Zulassen von Spontanatemversuchen des Patienten durchgeführt. Diese Art der Beatmung wurde zuvor unter Bezug auf die Figur 2 näher erläutert. Ferner wird bei Detektion des Vorliegens eines gewünschten Betriebszustandes hinsichtlich der automatisierten Beatmung ein Wechsel in einen zweiten Betriebsmodus M02 ermöglicht. Dieses ist vorzugsweise dann möglich, wenn der Betriebszustand innerhalb eines vorgegebenen Zeitraumes detektiert wird. Vorzugsweise erfolgt dann der Wechsel in den zweiten Betriebsmodus M02 automatisch. Vorzugsweise erfolgt eine Ausgabe eines Ausgabesignals, welches das Vorliegen des erwünschten ersten Betriebszustands indiziert. Vorzugsweise wird dann in den zweiten Betriebsmodus MO2 gewechselt, wenn dies durch eine Eingabe E des Klinikers initiiert wird. Vorzugsweise stellt die Eingabe E des Klinkers auch eine Auswahl dar, welche bestimmte Ausführungsform verschiedener möglicher Ausführungsformen M1, M2 oder M3 des zweiten Betriebsmodus MO2 dann durchgeführt wird. Die verschiedenen Ausführungsformen M1, M2, M3 sind jeweilige Formen automatisierter Beatmung, wobei in der ersten Ausführungsform M1 eine rein druckgesteuerte Beatmung ohne Zulassen von Spontanatemversuchen des Patienten durchgeführt wird, wobei ferner zweiten Ausführungsform M2 eine druckgesteuerte Beatmung mit Zulassen von Spontanatemversuchen des Patienten durchgeführt wird, und wobei ferner in der dritten Ausführungsform M3 eine rein druckunterstützende Beatmung im Rahmen von Spontanatemversuchen des Patienten durchgeführt wird.

In dem zweiten Betriebsmodus M02 ist eine Recheneinheit der die Beatmung durchführenden Vorrichtung dazu ausgebildet, eine Atemgasfördereinheit in Abhängigkeit eines erfassten Drucks und eines vorgegebenen Solldruckwertes anzusteuern, wobei die Recheneinheit ferner dazu ausgebildet ist, eine Anpassung des Solldruckwertes und eine Anpassung einer Beatmungsfrequenz in Abhängigkeit des erfassten Volumenstromes bzw. eines ermittelten Tidalvolumens und in Abhängigkeit der erfassten Kohlendioxidkonzentration bzw. einer ermittelten endexspiratorischen Kohlendioxidkonzentration vorzunehmen. Derartige Ausführungsformen M1, M2, M3 einer rein druckgesteuerten Beatmung, einer druckgesteuerten Beatmung mit Zulassen von Spontanatemversuchen sowie einer rein druckunterstützenden Beatmung sind detailliert dargelegt in der deutschen Patentanmeldung "Beatmungsvorrichtung und Verfahren zur automatisierten Beatmung eines Patienten", Anmelderin Drägerwerk AG & Co. KGaA, Erfinder Stefan Mersmann, Wilfried Buschke, Prof. Christoph Hörmann, eingereicht beim Deutschen Patent- und Markenamt am 02. Dezember 2015.

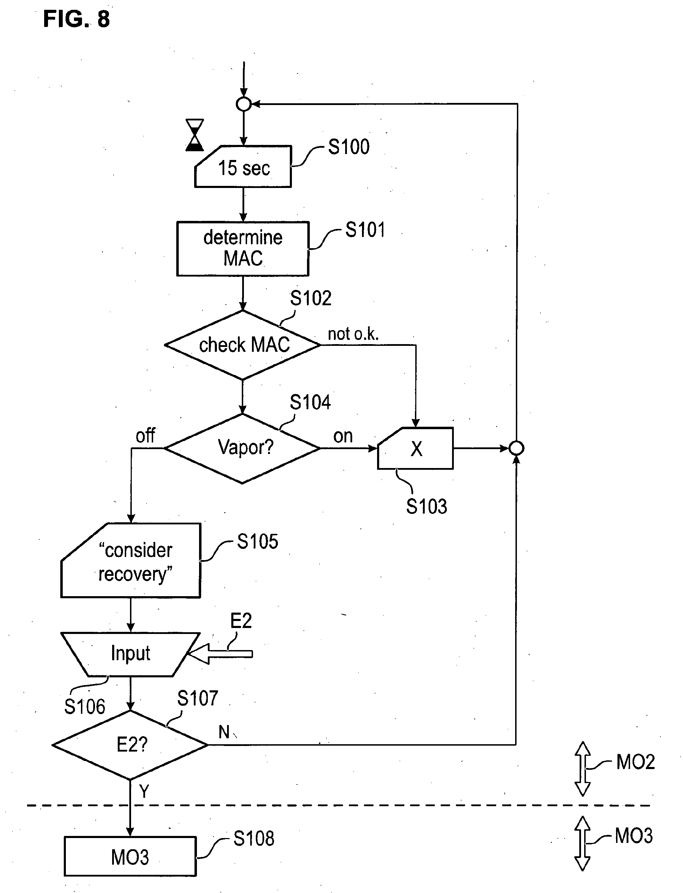

Wird nun in dem zweiten Betriebsmodus MO2 unter Verwendung eines anhand der Figur 8 später näher erläuterten Verfahrens detektiert, dass ein zweiter erwünschter Betriebszustand hinsichtlich der automatisierten Beatmung vorliegt, so wird vorzugsweise automatisch von dem zweiten Betriebsmodus MO2 in den dritten Betriebsmodus MO3 gewechselt. Vorzugsweise wird eine Ausgabe eines entsprechenden Ausgabesignals, welches dieses Vorliegen des zweiten erwünschten Betriebszustands indiziert, bewirkt. Vorzugsweise wird in Abhängigkeit einer weiteren Eingabe E2 des Klinkers von dem zweiten Betriebsmodus MO2 in den dritten Betriebsmodus MO3 gewechselt. In diesem dritten Betriebsmodus MO3 erfolgt eine reine druckunterstützende Beatmung im Rahmen von Spontanatemversuchen des Patienten.

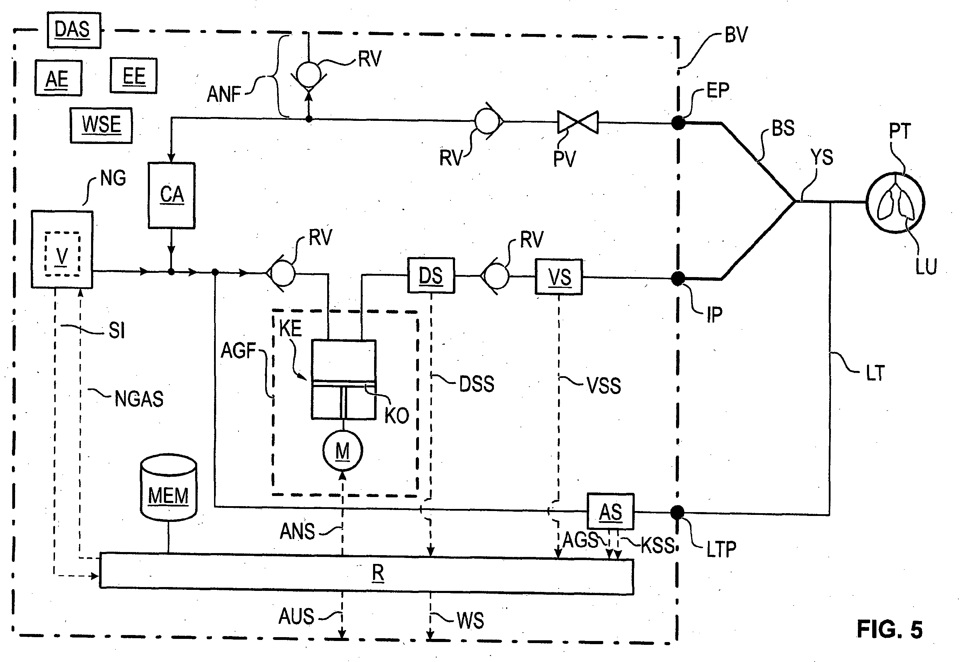

Die Figur 5 zeigt die erfindungsgemäße Anästhesiebeatmungsvorrichtung BV zur automatisierten Beatmung eines Patienten PT. Die erfindungsgemäße Anästhesiebeatmungsvorrichtung BV weist einen inspiratorischen Port IP und einen exspiratorischen Port EP auf, an welchen ein Beatmungsschlauch BS angeschlossen werden kann, welcher dem Patienten PT zugewandt ist. Über diesen Beatmungsschlauch BS wird ein Atemgas dem Patienten zugeführt und auch wieder von dem Patienten hin zur Vorrichtung BV abgeführt. Die Zuführung erfolgt über den inspiratorischen Port IP, die Abführung erfolgt über den exspiratorischen Port EP. Der Beatmungsschlauch BS führt die Anschlüsse der Ports EP, IP an einem sogenannten Y-Stück YS zusammen, welches dann üblicherweise an einem Tubus endet, der dem Patienten PT eingeführt wird, um ihn über seine Lunge LU zu beatmen.

Die Anästhesiebeatmungsvorrichtung BV weist ferner eine Atemgasfördereinheit AGF auf. Vorzugsweise ist die Atemgasfördereinheit AGF eine Kolbeneinheit KE, in welcher ein Kolben KO durch einen Motor M vor- bzw. zurückgeführt werden kann.

Die Vorrichtung BV weist ferner wenigstens eine Recheneinheit R auf, welche auch durch einen Verbund mehrerer Recheneinheiten verwirklicht werden kann. Die Recheneinheit R ist dazu ausgebildet, über ein Ansteuersignal ANS die Atemgasfördereinheit AGF anzusteuern.

Ferner weist die Anästhesiebeatmungsvorrichtung BV einen Drucksensor DS zum Erfassen eines Drucks des Atemgases auf. Der Drucksensor DS stellt ein Drucksensorsignal DSS an die Recheneinheit R bereit.

Die Beatmungsvorrichtung BV weist wenigstens einen Volumenstromsensor VS zum Erfassen eines Volumenstroms des Atemgases auf. Der Volumenstromsensor VS stellt ein Volumenstromsensorsignal VSS an eine Recheneinheit R bereit.

Ein Mindestdruck PEEP wird vorzugsweise durch ein Ventil PV, welches vorzugsweise in dem Bereich des exspiratorischen Ports EP liegt, realisiert.

Die Anästhesiebeatmungsvorrichtung BV weist ferner wenigstens einen Atemgassensor AS auf. Der Sensor AS ist vorzugsweise hinter einer Messleitung LT vorgesehen, welche eine Messprobe des Atemgases am Y-Stück YS abführt und an einen Messgasport LTP angeschlossen ist.

Der wenigstens eine Atemgassensor AS ist ausgebildet zum Erfassen einer Kohlendioxidkonzentration des Atemgases. Der Atemgassensor stellt ein Kohlendioxidkonzentrationssignal KSS an die Recheneinheit bereit. Der wenigstens eine Atemgassensor AS ist ferner vorzugsweise ausgebildet zum Erfassen einer Anästhesiegaskonzentration des Atemgases. Der Atemgassensor stellt vorzugsweise ein Anästhesiegaskonzentrationssignal AGS an die Recheneinheit R bereit. Der wenigstens eine Atemgassensor AS ist vorzugsweise kein einzelner Sensor sondern eine Sensoreinheit AS. Eine Solche Sensoreinheit AS weist mehrere, jeweils speziell ausgebildete Sensoren zur Erfassung der jeweiligen zuvor genannten Konzentrationen auf.

Die Anästhesiebeatmungsvorrichtung BV weist einen Kohlendioxidabsorber CA sowie eine Narkosegasmischeinheit NG auf. Die Narkosemischeinheit NG weist vorzugsweise einen Vapor V auf. Über die Narkosegasmischeinheit NG kann dann ein für die Narkose erforderliches Gasgemisch in den Atemkreislauf eingebracht werden. Ein solches Gasgemisch weist dann wenigstens ein Anästhetikum auf.

Ferner weist die Anästhesiebeatmungsvorrichtung eine Anästhesiegasfortleitung ANF bzw. einen Anschluss an eine Anästhesiegasfortleitung ANF auf.

Die Recheneinheit R steuert die Narkosegasmischeinheit NG mittels eines Steuersignals NGAS. Die Narkosemischeinheit NG stellt vorzugsweise ein Statussignal SI an die Recheneinheit R bereit, welches indiziert, ob die Narkosemischeinheit NG ein Anästhetikum in das Atemgas einbringt oder nicht. Vorzugsweise indiziert dieses Statussignal SI, ob der Vapor V geöffnet ist oder nicht.

Der Gasfluss innerhalb der Anästhesiebeatmungsvorrichtung BV ist vorzugsweise durch Rückschlagventile RV kontrolliert.

Die Anästhesiebeatmungsvorrichtung BV aus Figur 5 weist eine Eingabeeinheit EE bzw. eine Schnittstelle zu einer Eingabeeinheit EE auf, mittels welcher Eingaben an der Anästhesiebeatmungsvorrichtung BV entgegengenommen werden können, welche durch einen Bediener bzw. Kliniker vorgenommen werden können.

Die Recheneinheit R greift vorzugsweise auf eine Speichereinheit MEM zu, um das nicht von der Erfindung umfasste Verfahren durchzuführen.

Die Recheneinheit R gibt vorzugsweise im Zuge einer rein druckunterstützenden Beatmung ein Warnsignal WS aus, um ein Vorliegen einer Beatmungsfrequenz, welche eine minimale Beatmungsfrequenz unterschreitet, zu indizieren. Diese Ausgabe erfolgt vorzugsweise über eine Datenschnittstelle DAS der Vorrichtung BV. Vorzugsweise weist die Vorrichtung BV selber eine Warnsignalausgabeeinheit WSE auf, welche vorzugsweise eine optische und/oder akustische Warnung ausgeben kann.

Die Recheneinheit R gibt vorzugsweise ein Ausgabesignal AUS aus, welches ein Vorliegen eines detektierten Betriebszustandes indiziert. Diese Ausgabe erfolgt vorzugsweise über die Datenschnittstelle DAS hin zu einer nicht gezeigten Anzeigeeinheit. Vorzugsweise erfolgt die Ausgabe über eine Anzeigeeinheit AE, welche Teil der Vorrichtung BV ist.

Die erfindungsgemäße Anästhesiebeatmungsvorrichtung BV ist dazu ausgebildet, in einem ersten Betriebsmodus eine rein druckgesteuerte Beatmung des Patienten PT durchzuführen. Ferner ist die erfindungsgemäße Anästhesiebeatmungsvorrichtung BV dazu ausgebildet, in einem zweiten Betriebsmodus eine Beatmung nach einer der Ausführungsformen M1, M2 oder M3, wie unter Bezug auf Figur 13 beschrieben, durchzuführen.

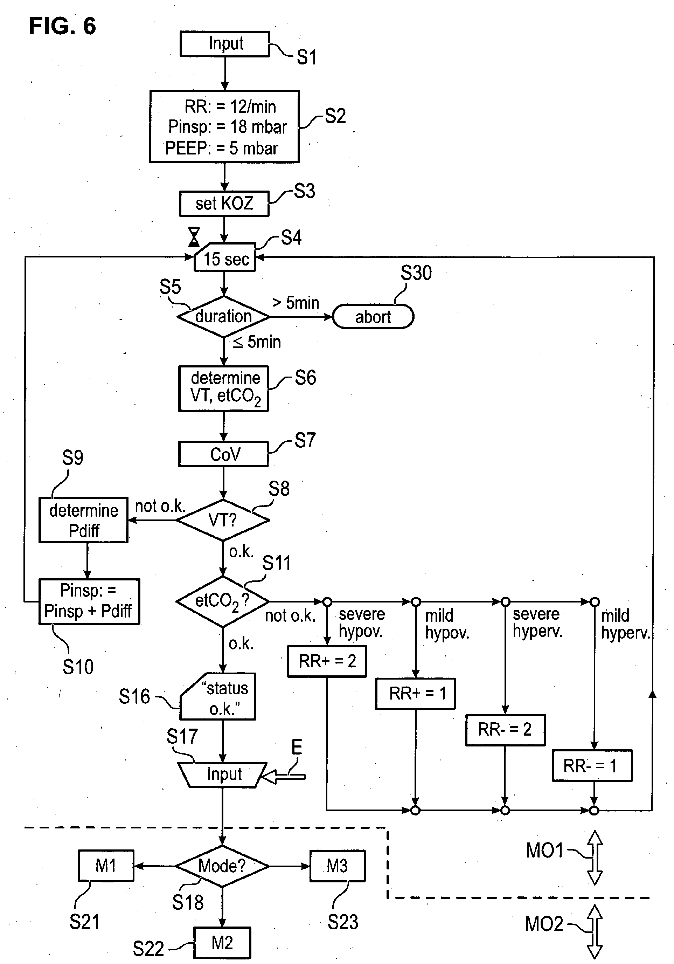

Figur 6 zeigt Initiationsschritte, mittels derer die Anästhesiebeatmungsvorrichtung BV aus Figur 5 zur Durchführung des nicht von der Erfindung umfassten Verfahrens veranlasst werden kann. In einem Schritt S1 erfolgt eine Eingabe von Vorgaben durch einen Nutzer bzw. Kliniker.

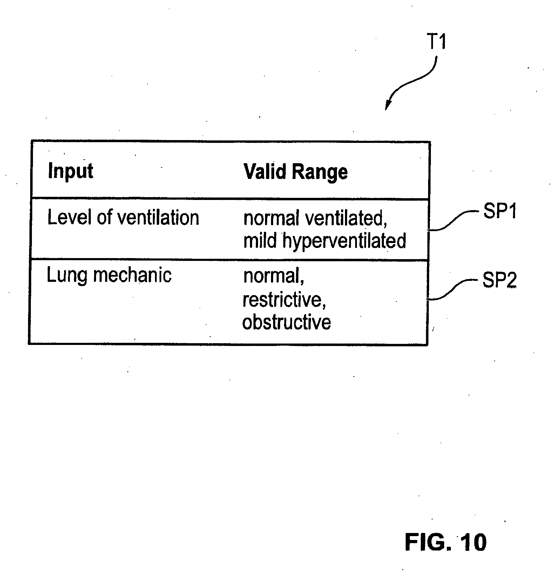

Figur 10 zeigt in der Tabelle T1 Möglichkeiten verschiedener möglicher Eingaben bzw. Vorgaben im Rahmen des Schrittes S1 der Figur 6. In der ersten Spalte SP1 finden sich verschiedene Einträge bzw. Vorgaben, mittels derer ein gewünschter Ventilationsgrad bzw. eine gewünschte Gasaustauchrate eines Patienten ausgewählt werden kann. Hierbei handelt es sich vorzugsweise um die Varianten einer normalen Beatmung bzw. einer milden Hyperventilation. Derartige Eingaben können durch die in Figur 5 gezeigte Schnittstelle EE bzw. Eingabeeinheit EE der Anästhesiebeatmungsvorrichtung BV entgegengenommen werden. Diese Eingabeeinheit EE kann vorzugsweise eine zu der Anästhesiebeatmungsvorrichtung BV zugehörige oder eine mit der Anästhesiebeatmungsvorrichtung BV in Kommunikation stehende Eingabeeinheit in Form einer Tastatur, eines Touch-Screens und/oder einer Computermaus sein.

In Figur 10 finden sich in der zweiten Spalte SP2 ferner verschiedene Möglichkeiten von Eingaben bzw. Vorgaben bezüglich einer Lungeneigenschaft ("Lung Mechanic") des Patienten. Die Vorgabe kann eine Lungeneigenschaft einer normalen, einer restriktiven oder einer obstruktiven Lunge indizieren.

Zurückkommend zur Figur 6 kann nun in dem Schritt S2 eine Initialisierung von beatmungsrelevanten Parametern vorgenommen werden. Hierbei wird die Beatmungsfrequenz RR vorzugsweise auf 12 Atemzüge/min gesetzt, der Mindestdruck PEEP auf 5 mbar sowie der maximale Druckwert Pinsp auf 18 mbar. Es ist für einen Fachmann klar, dass die hier gezeigten Werte nur exemplarische Werte sind und bei Ausführung des nicht von der Erfindung umfassten Verfahrens sowie Realisierung der erfindungsgemäßen Vorrichtung auch anders gewählt werden können.

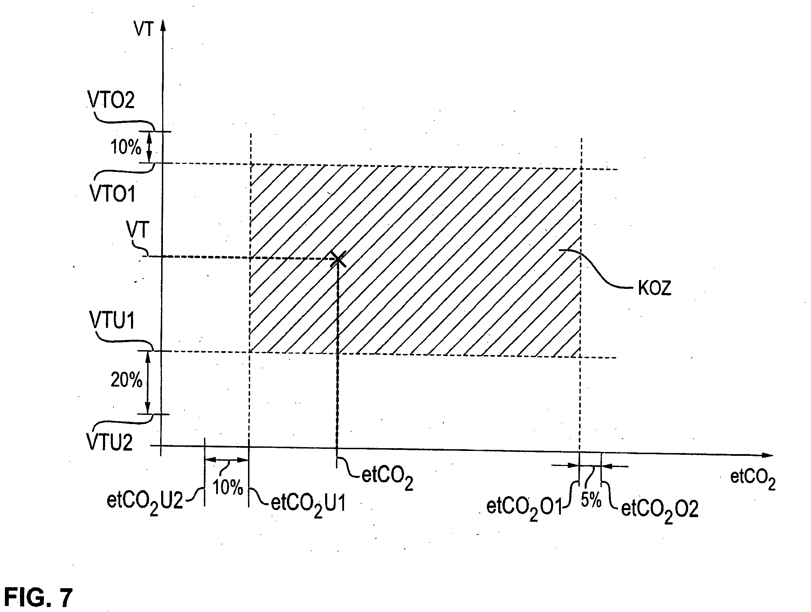

In einem Verfahrensschritt S3 werden Grenzwerte definiert, welche vorzugsweise eine sogenannte Komfortzone KOZ indizieren. Hierzu lässt sich in Korrespondenz zu diesem Schritt S3 die Figur 7 betrachten.

Die Figur 7 indiziert die Komfortzone KOZ, welche immer dann gegeben ist, wenn aus dem erfassten Volumenstrom durch die Recheneinheit ein durch den Patienten eingeatmetes Tidalvolumen VT ermittelt wird, welches zwischen einem oberen Volumengrenzwert VTO1 und einem unteren Volumengrenzwert VTU1 liegt. Ferner ist es zum Erreichen der Komfortzone KOZ erforderlich, dass die durch Recheneinheit R auf Basis des Kohlendioxidkonzentrationssignals KSS ermittelte endexspiratorische Kohlendioxidkonzentration etCO2 ermittelt wird, welche zwischen einem oberen Konzentrationsgrenzwert etCO2O1 und einem unteren Konzentrationsgrenzwert etCO2U1 liegt.

Ein vorzugsweise zu erreichendes Ziel des nicht von der Erfindung umfassten Verfahrens ist es, den Patienten derart zu beatmen, dass durch die Beatmung der Patient ein Tidalvolumen VT aufweist bzw. atmet, welches innerhalb der Volumengrenzwerte VTO1, VTU1 liegt, und gleichzeitig auch eine endexspiratorische Kohlendioxidkonzentration etCO2 erzeugt, welche innerhalb der Konzentrationsgrenzwerte etCO2U1 und etCO2O1 liegt.

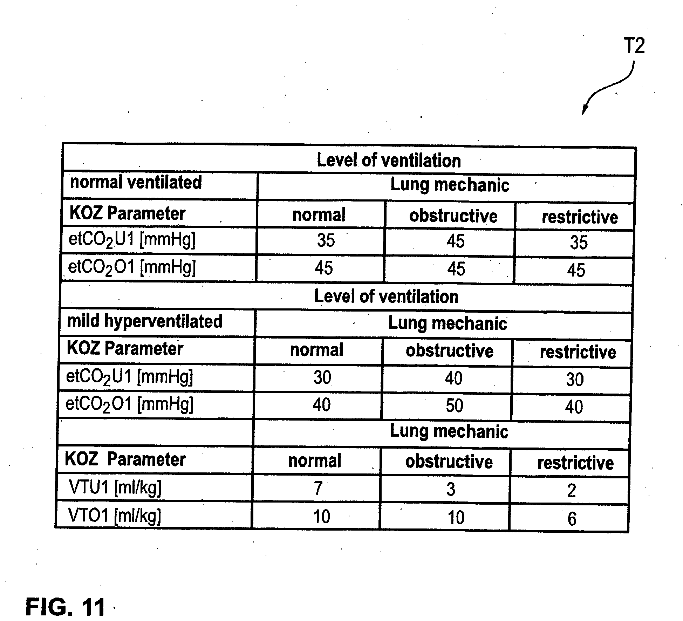

In Bezug auf den Schritt S3 aus Figur 6 kann unter Hinzunahme der Figur 11 und unter Betrachtung der Tabelle T2 entnommen werden, in welcher Weise durch Auswahl der Vorgaben hinsichtlich der Lungeneigenschaft ("Lung mechanic") des Patienten sowie der Gasaustauchrate bzw. des Ventilationsgrades ("normal ventilated" / "mildhyperventilated") die jeweiligen Volumengrenzwerte VTO1, VTU1 sowie die jeweiligen Konzentrationsgrenzwerte etCO2U1, etCO2O1 gewählt werden können.

Die Recheneinheit R der Figur 5 ermittelt auf Basis des erfassten Volumenstroms ein durch den Patienten eingeatmetes Tidalvolumen VT. Hierzu bestimmt die Recheneinheit vorzugsweise anhand des erfassten Volumenstroms und der Dauer Ti der inspiratorischen Phase das Tidalvolumen als Integralwert des Volumenstroms über diese Dauer Ti. Alternativ kann eine Zeitdauer einer inspiratorischen Phase derart ermittelt werden, dass bei Überschreiten eines Volumenstromschwellenwertes auf einen Beginn der inspiratorischen Phase geschlossen wird und bei Unterschreiten des Volumenstromschwellenwertes auf ein Ende der inspiratorischen Phase.

Die Recheneinheit bestimmt auf Basis der erfassten Kohlendioxidkonzentration eine endexspiratorische Kohlendioxidkonzentration etCO2. Vorzugsweise wird durch Vergleich des Volumenstroms, wie in Figur 1 gezeigt, und eines unteren bzw. negativen USW Schwellenwertes dann auf ein Ende einer endexspiratorischen Phase geschlossen, wenn der erfasste Volumenstrom den unteren Schwellenwert von unten her durchschreitet.

Die durch die Recheneinheit R der Figur 5 bestimmten Werte für das Tidalvolumen sowie die endexspiratorische Kohlendioxidkonzentration werden vorzugsweise alle 4 Sekunden als Messwerte bereitgestellt.

Gemäß des Schrittes S4 der Figur 6 wird zunächst 15 Sekunden lang gewartet.

In dem Verfahrensschritt S5 wird dann überprüft, ob der erste Betriebsmodus MO1 bereits für einen maximalen Zeitraum von vorzugsweise mehr als 5 Minuten oder weniger als der maximale Zeitraum andauert. Ist der vorgegebene Maximalzeitraum von vorzugsweise 5 Minuten überschritten, so führt das Verfahren weiter zu einem Verfahrensschritt S30, in welchem das Verfahren vorzugsweise abgebrochen wird. Ist also während eines vorgegebenen maximalen Zeitraums, vorzugsweise 5 Minuten, kein erwünschter Betriebszustand hinsichtlich der automatisierten Beatmung detektiert worden, so wird kein Übergang von dem ersten Betriebsmodus M01 in den zweiten Betriebsmodus M02 ermöglicht. Es wird vorzugsweise durch die Recheneinheit ein Ausgabesignal ausgegeben, welches indiziert, dass innerhalb des maximalen Zeitraumes der erwünschte Betriebszustand nicht erreicht werden konnte. Dieses kann dann ggf. durch den Kliniker als Hinweis darauf gewertet werden, dass der Patient innerhalb des vorgegebenen Zeitraumes kein adäquates Atemverhalten aufweist, um stabil mittels des zweiten Betriebsmodus beatmet zu werden.

Dauert der erste Betriebsmodus MO1 weniger als der vorgegebene Zeitraum von vorzugsweise 5 Minuten an, so wird zu dem Verfahrensschritt S6 fortgeführt.

Es wird dann im Rahmen des Schrittes S6 das in Betracht gezogene Tidalvolumen VT mittels einer Vorverarbeitung, vorzugsweise einer Medianfilterung, jener Messwerte des Tidalvolumens ermittelt, welche in den letzten 60 Sekunden vorlagen bzw. gemessen wurden. Ebenso wird die in Betracht gezogene endexspiratorische Kohlendioxidkonzentration etCO2 mittels einer Vorverarbeitung, vorzugsweise einer Medianfilterung, anhand jener Messwerte der endexspiratorischen Kohlendioxidkonzentration ermittelt, welche die letzten 60 Sekunden repräsentieren bzw. innerhalb der letzten 60 Sekunden gemessen wurden.

Nach Bestimmung des Tidalvolumens VT sowie der endexspiratorischen Kohlendioxidkonzentration etCO2 wird im Rahmen des Schrittes S7 ein Ventilationsgrad unter Bezug auf das Tidalvolumen bestimmt sowie ferner ein Ventilationsgrad in Bezug auf die endexspiratorische Kohlendioxidkonzentration.

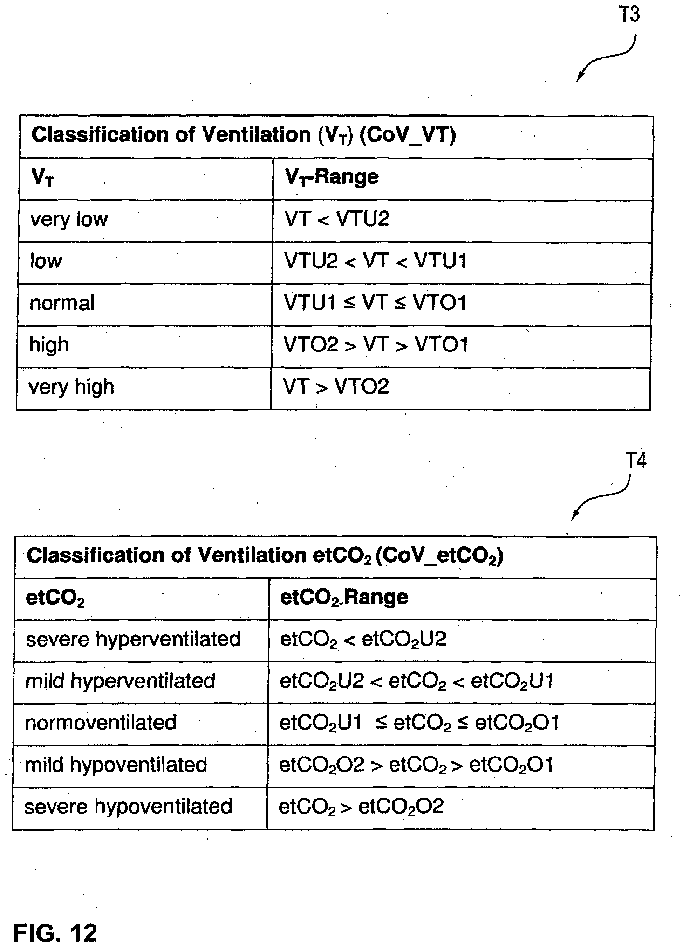

Hierzu ist die Figur 12 heranzuziehen, welche in der Tabelle T3 verschiedene Ventilationsgrade unter Vergleich des Tidalvolumens VT zu den zuvor ermittelten Grenzwerten vorgibt. Hierbei werden nicht nur der obere und der untere Grenzwert VTO1, VTU1, wie zuvor in Tabelle T2 gezeigt, herangezogen, sondern auch weitere, zweite Grenzwerte VTO2, VTU2. Diese zweiten Grenzwerte VTO2, VTU2 sind ebenso in Figur 7 eingetragen. Es werden also jeweilige zweite Grenzwerte VTO2, VTU2 verwendet, bei welchen eine Abweichung um 10% bzw. 20% von den ersten Grenzwerten VTO1, VTU1 in Betracht gezogen wird.

Entsprechendes gilt für den Grad einer Ventilation in Bezug auf die endexspiratorische Kohlendioxidkonzentration in Vergleich zu den Konzentrationswerten etCO2U1, etCO2O1 sowie weiteren zweiten Konzentrationswerten etCO2U2, etCO2O2, welche um 5% bzw. 10% von den ersten Konzentrationswerten etCO2U1, etCO2O1 abweichen und ebenfalls in Figur 7 eingetragen sind. Anhand der Tabelle T4 der Figur 12 kann also ein Ventilationsgrad in Bezug auf die endexspiratorische Kohlendioxidkonzentration bestimmt werden.

In dem Verfahrensschritt S8 wird dann überprüft, ob das ermittelte Tidalvolumen VT sich innerhalb der Volumengrenzwerte befindet. Mit anderen Worten, es wird überprüft, ob das gemessene Tidalvolumen zwischen dem oberen Grenzwert VTO2 und dem unteren Grenzwert VTU1 liegt. Ist dies nicht der Fall, so wird zu einem Verfahrensschritt S9 verzweigt, in welchem dann gemeinsam mit dem Verfahrensschritt S10 der Solldruckwert Pinsp angepasst wird.

In dem Verfahrensschritt S9 wird eine gewünschte Druckänderung Pdiff ermittelt. Mit dieser Druckänderung Pdiff kann ggf. ein für den jeweiligen Patienten geeignetes Minutenvolumen MV

$$MV = RR * VT$$

erreicht werden.

Dieses erfolgt derart, dass ein Zieltidalvolumen TVT vorgegeben wird. Dieses ist vorzugsweise abhängig von dem oberen Volumengrenzwert VTO1 und dem unteren Volumengrenzwert VTU1 gemäß

$$TVT := (VTO1 - VTU1) / 2$$

Auf Basis eines vorgegebenen Druckanpassungswertes DEP, dem Zieltidalvolumen TVT sowie dem gemessenen Tidalvolumen VT kann nun die gewünschte Druckänderung Pdiff in dem Schritt S10 bestimmt werden. Dies erfolgt gemäß

$$Pdiff := ((TVT - VT) * DEP) / VT \quad .$$

In dem Schritt S10 erfolgt dann die Anpassung des Solldruckwertes Pinsp, vorzugsweise gemäß

$$Pinsp := Pinsp + Pdiff \quad .$$

Es wird dann zurückgekehrt zu dem Verfahrensschritt S4.

Ergab die Überprüfung in dem Verfahrensschritt S8, dass das gemessene Tidalvolumen VT sich bereits innerhalb der vorgegebenen Volumengrenzwerte, also innerhalb der Komfortzone Korridors KOZ, befand, so wird weiter verfahren zu einem Verfahrensschritt S11, in welchem dann die endexspiratorische Kohlendioxidkonzentration etCO2 überprüft wird.

Ergab die Überprüfung in dem Verfahrensschritt S11, dass die endexspiratorische Kohlendioxidkonzentration etCO2 nicht innerhalb der Konzentrationsgrenzwerte bzw. der Komfortzone KOZ aus Figur 7 lag, so wird zu den weiteren Verfahrensschritten S12 bis S15 verzweigt, welche eine Anpassung der Atemfrequenz RR vornehmen. Mit anderen Worten, liegt die gemessene endexspiratorische Kohlendioxidkonzentration etCO2 nicht zwischen dem unteren Kohlendioxidgrenzwert etCO2U1 und dem oberen Kohlendioxidgrenzwert etCO2O1, so erfolgt eine Anpassung der Beatmungsfrequenz RR.

Es wird dann bei Verzweigung von dem Schritt S11 hin zu den Schritten S12 bis S15 zunächst unter Zuhilfenahme der Tabelle T4 aus Figur 12 ermittelt, ob ein Hyperventilationsgrad, ein normaler Ventilationsgrad oder ein Hypoventilationsgrad des Patienten vorliegt. Aufgrund der Einträge in der Tabelle T4 kann der entsprechende Zustand des Patienten ermittelt werden. Dieser Zustand hängt von der gemessenen endexspiratorischen Kohlendioxidkonzentration etCO2 sowie der entsprechenden Grenzwerte etCO2U2, etCO2U1, etCO2O1, etCO2O2 ab.

Liegt eine starke Hypoventilation ("severe hypoventilated") vor, so erfolgt in dem Verfahrensschritt S12 eine Anpassung der Beatmungsfrequenz RR derart, dass diese um den Wert von 2 pro Minute erhöht wird.

Entsprechende Anpassungen in Abhängigkeit der entsprechenden Ventilationsgrade milder Hypoventilation, milder Hyperventilation sowie starker Hyperventilation erfolgen entsprechen in den alternativen Verfahrensschritten S13, S14 oder S15. Nach Anpassung der Beatmungsfrequenz in einem der Schritte S12 bis S15 wird zurück verzweigt zu dem Verfahrensschritt S4.

Hat die Überprüfung des Tidalvolumens VT in dem Verfahrensschritt S8 sowie die Überprüfung der endexspiratorischen Kohlendioxidkonzentration etCO2 in dem Verfahrensschritt S11 ergeben, dass beide Werte innerhalb der der entsprechenden Grenzwerte VT_O1, VT_U1, etCO2U1, etCO2O1 bzw. innerhalb der Komfortzone KOZ liegen, so kann angenommen werden, dass der erwünschte Betriebszustand hinsichtlich der automatisierten Beatmung vorliegt und dieser wird somit durch Übergang von dem Schritt S11 hin zu den weiteren Schritten S16 etc. detektiert. Es wird dann von dem Verfahrensschritt S11 hin zu dem Verfahrensschritt S16 verzweigt, in welchem vorzugsweise ein Ausgabesignal ("Status o.k.") ausgegeben wird, welches das Vorliegen des erwünschten Betriebszustands indiziert.

In einem Verfahrensschritt S17 wird dann vorzugsweise auf ein Eingabesignal E eines Klinikers gewartet. Liegt diese Eingabe E des Klinikers vor, so wird dann vorzugsweise in einen zweiten Betriebsmodus MO2 gewechselt. Vorzugsweise erfolgt der Übergang von dem Schritt S11 hin zu dem Schritt S18 ohne eine Überprüfung auf ein Eingabesignal E in dem Schritt S17.

Vorzugsweise indiziert die Eingabe E eine Auswahl einer bevorzugten Ausführungsform M1, M2, oder M3 einer entsprechenden Beatmungsform, welche zuvor unter Bezug auf Figur 13 näher erläutert wurden. In dem zweiten Betriebsmodus MO2 kann dann vorzugsweise in einem Unterschritt S18 abhängig von der Eingabe E des Klinikers zu einer der Ausführungsformen M1, M2 oder M3 bzw. der entsprechenden Schritte S21, S22 oder S23 verzweigt werden.

In dem zweiten Betriebsmodus MO2 kann ferner parallel zu einem Beatmungsverfahren gemäß einer der Ausführungsformen M1, M2 oder M3 ein Detektionsverfahren durchgeführt werden, welches nun unter Bezug auf die Figur 8 näher erläutert wird.

Die Figur 8 zeigt Teilverfahrensschritte, welche ein nicht von der Erfindung umfasstes Verfahren illustrieren, welches im Zuge des zweiten Betriebsmodus fortlaufend durchgeführt werden kann, um das Vorliegen eines zweiten erwünschten Betriebszustand hinsichtlich der automatisierten Beatmung zu detektieren. Hierbei handelt es sich vorzugsweise um einen Betriebszustand, welcher für einen Kliniker ein Hinweis darauf sein kann, dass der Patient eine aus Sicht des Klinikers stabile Atemaktivität aufweist, so dass der Kliniker ein Herbeiführen eines dritten Betriebsmodus mit einer druckunterstützenden Beatmung herbeiführen kann, vorzugsweise durch eine weitere Eingabe E2.

Für die Detektion des zweiten Betriebszustandes wird eine Anästhesiegaskonzentration des Atemgases in Betracht gezogen.

In einem ersten Teilverfahrensschritt S100 wird zunächst für einen Zeitraum von 15 Sekunden abgewartet.

In einem Teilverfahrensschritt S101 wird dann eine mittlere alveoläre Anästhesiegaskonzentration bestimmt. Eine solche mittlere alveoläre Anästhesiegaskonzentration wird auch "minimum alveolar concentration" genannt. Diese mittlere alveoläre Anästhesiegaskonzentration MAC ist vorzugsweise eine normierte Größe xMAC, bzw. ein MAC Vielfaches, wie in der Schrift

- "Primus Infinity Empowered", Dräger Medical GmbH, Ausgabe/Edition: 3/2010/09, Seiten 132 - 134,

offenbart.

Eine solche mittlere alveoläre Anästhesiegaskonzentration MAC wird über ein gemitteltes Zeitfenster zurückliegender Messwerte, welche durch das Anästhesiegaskonzentrationssignal AGS indiziert werden, siehe Figur 5, ermittelt.

Hierzu bestimmt die Recheneinheit R auf Basis des Anästhesiegaskonzentrationssignals AGS der Figur 5 vorzugsweise während einer endexspiratorischen Phase die Konzentration des Anästhesiegases. Hierzu kann die Recheneinheit R vorzugsweise ein Vorliegen einer exspiratorischen Phase EXP aus Figur 1 unter zu Hilfenahme des unteren Schwellenwertes USW in der zuvor genannten bzw. beschriebenen Weise heranziehen.

In dem Teilschritt S102 der Figur 8 wird dann überprüft, ob ein zeitlicher Verlauf der bestimmten mittleren, alveolären Anästhesiegaskonzentration über der Zeit vorgegebene Bedingungen erfüllt.

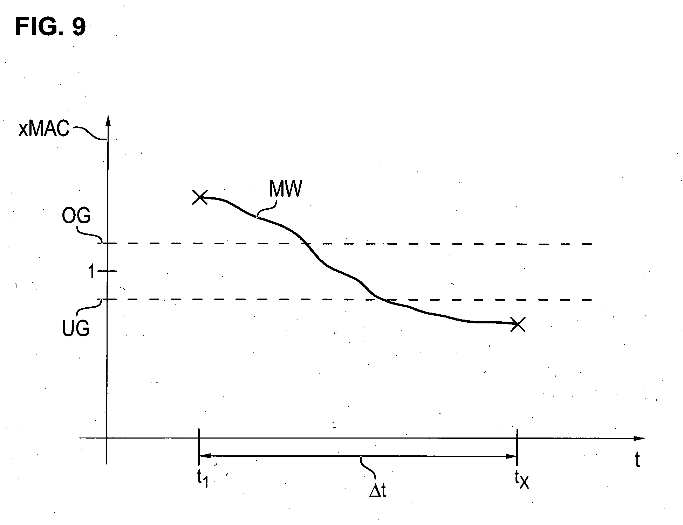

Hierzu illustriert Figur 9 eine solche Überprüfung des zeitlichen Verlaufs der mittleren alveolären Anästhesiegaskonzentration MAC über der Zeit t. Messwerte MW werden für einen aktuellen Zeitpunkt tX sowie einen um ein Zeitintervall Δt zurückliegenden Zeitpunkt t1 betrachtet. Bei der mittleren alveolären Anästhesiegaskonzentration MAC handelt es sich vorzugsweise um die zuvor erwähnte Anästhesiegaskonzentration xMAC.

Liegt für den zurückliegenden Zeitpunkt t1 die mittlere alveoläre Anästhesiegaskonzentration xMAC oberhalb eines oberen Grenzwertes OG, welcher vorzugsweise der Wert 1,1 ist, und liegt die mittlere alveoläre Anästhesiegaskonzentration xMAC unterhalb eines unteren Grenzwertes UG, welcher vorzugsweise der Wert 0,9 ist, so wird ein adäquates Abnehmen der mittleren alveolären Anästhesiegaskonzentration xMAC angenommen. Dieses ist eine zeitliche Abnahme der mittleren alveolären Anästhesiegaskonzentration, wie sie bei einer Ausleitung einer Anästhesiebeatmungsphase zu erwarten ist, bei welcher durch die Narkosegaseinheit NG der Figur 5 keine weiteren Anästhesiegase in das Atemgas eingebracht werden.

Erfüllt die mittlere alveoläre Anästhesiegaskonzentration in dem Teilverfahrensschritt S102 über der Zeit die erforderten Bedingungen, so wird hin zu einem Verfahrensschritt S104 verzweigt, in welchem ferner ein Betriebszustand des Vapors als Teil der Narkosemischeinheit NG der Figur 5 überprüft wird.

Ist der Vapor noch eingeschaltet, so ist nicht unbedingt zu erwarten, dass tatsächlich eine Beendigung der Narkose des Patienten vorgenommen werden soll, sodass das Verfahren dann von dem Schritt S104 hin zu einem Verfahrensschritt S103 verzweigt.

In dem Verfahrensschritt S103 wird eine möglicherweise ausgegebene Meldung, wie beispielsweise die Meldung ("Consider Recovery") aus Verfahrensschritt S105, zurückgenommen. Auch in dem Fall, dass in dem Verfahrensschritt S102 die Überprüfung der mittleren alveolären Anästhesiegaskonzentration nicht zufriedenstellend war, wird direkt von dem Verfahrensschritt S102 hin zu dem Verfahrensschritt S103 verzweigt.

Von dem Verfahrensschritt S103 wird zurückgekehrt zu dem Verfahrensschritt S100.

Wird in dem Verfahrensschritt S104 detektiert, dass der Vapor ausgestaltet ist, so wird dann darauf geschlossen, dass der zweite Betriebszustand vorliegt und dieser wird somit detektiert. Daher wird dann in dem Verfahrensschritt S105 vorzugsweise ein Ausgabesignal ausgegeben, welche indiziert, dass das Vorliegen des zweiten, erwünschten Betriebszustands gegeben ist ("Consider Recovery"). Es kann also eine sogenannte Erholungsphase oder Recovery-Phase des Patienten durch den Kliniker in Betracht gezogen werden. In einer solchen Erholungs- bzw. Recovery-Phase wird dann vorzugsweise als dritter Betriebsmodus MO3 eine rein druckunterstützte Beatmung durchgeführt.

Erfolgt vorzugsweise in dem Verfahrensschritt S106 die Eingabe eines weiteren Eingabesignals E2 durch den Kliniker, so wird dann in dem Schritt S107 in den dritten Betriebsmodus MO3 bzw. den Verfahrensschritt S108 fortgeschritten.

Vorzugsweise erfolgt bei Detektion des zweiten Betriebszustandes ein Wechsel von dem zweiten Betriebsmodus M02 zu dem dritten Betriebsmodus M03 automatisch ohne Abhängigkeit eines Eingabesignals E2 durch einen Nutzer bzw. Kliniker. Daher wird dann also direkt von dem Schritt S104 hin zu dem Schritt S108 verfahren.

In dem dritten Betriebsmodus MO3 steuert die Recheneinheit die Atemgasfördereinheit in Abhängigkeit des erfassten Drucks und eines vorgegebenen Solldruckwerts im Rahmen einer reinen druckunterstützenden Beatmung an. Hierbei nimmt die Recheneinheit eine Anpassung des Solldruckwertes ΔP in Abhängigkeit der erfassten Anästhesiegaskonzentration vor. In welcher Weise die Recheneinheit die Anpassung des Solldruckwertes in Abhängigkeit der erfassten Anästhesiegaskonzentration vornehmen kann, ist in der Anmeldung Anästhesiebeatmungsvorrichtung zur automatisierten Beatmung eines Patienten, Anmelderin Drägerwerk AG & Co. KGaA, Erfinder Stefan Mersmann, Wilfried Buschke, Prof. Christoph Hörmann, eingereicht beim Deutschen Patent- und Markenamt am 02. Dezember 2015 im Detail erläutert.

Obwohl manche Aspekte im Zusammenhang mit einer Vorrichtung beschrieben wurden, versteht es sich, dass diese Aspekte auch eine Beschreibung des entsprechenden nicht von der Erfindung umfassten Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks/Schrittes oder Details oder Merkmals einer entsprechenden Vorrichtung dar, bzw. dass die Vorrichtung oder die entsprechende Recheneinheit zur Durchführung des Verfahrensschrittes ausgebildet ist.

Die in Figur 5 gezeigte Recheneinheit R ist als wenigstens eine Recheneinheit anzusehen. Eine Umsetzung der wenigstens einen Recheneinheit R kann auch durch eine Kombination mehrerer Recheneinheiten verwirklicht werden, vorzugsweise durch Verwendung von Software in Verbindung mit Hardware. Je nach bestimmten Implementierungsanforderungen können Ausführungsbeispiele der Erfindung in Hardware und/oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardware-Komponente derart zusammenwirken können oder zusammenwirken, dass das jeweilige Verfahren durchgeführt wird.

Eine programmierbare Hardware-Komponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardware-Komponente derart zusammenzuwirken, dass eines der hierin beschriebenen nicht von der Erfindung umfassten Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen nicht von der Erfindung umfassten Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardware-Komponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen nicht von der Erfindung umfassten Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der nicht von der Erfindung umfassten Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

**Bezugszeichenliste**

| | |
|---|---|
| Anzeigeeinheit | AE |
| Anästhesiegasfortleitung | ANF |
| Atemgasfördereinheit | AGF |
| Ausgabesignal | AGS |
| Ansteuersignal | ANS |
| Atemgassensor | AS |
| Beatmungsschlauch | BS |
| Beatmungsvorrichtung | BV |
| Kohlendioxidabsorber | CA |
| Änderung des Druckwertes $\Delta P$ | dP |
| Änderungsgrad der Beatmungsfrequenz RR | dRR |
| Datenschnittstelle | DS |
| Drucksensor | DAS |

(fortgesetzt)

| | |
|---|---|
| Drucksensorsignal | DSS |
| Eingabeeinheit | EE |
| Exspiratorische Phase | EXP |
| Exspiratorischer Port | EP |
| endexspiratorischen Kohlendioxidkonzentration | etCO2 |
| oberer Konzentrationsgrenzwert | etCO2O1, etCO2O2 |
| unterer Konzentrationsgrenzwert | etCO2U1, etCO2U2 |
| Triggerschwelle | FT |
| Inspiratorische Phase | INP |
| Inspiratorischer Port | IP |
| Kolbeneinheit | KE |
| Kolben | KO |
| Komfortzone | KOZ |
| Kohlendioxidkonzentrationssignal | KSS |
| Messgasleitung | LT |
| Messgasport | LTP |
| Motor | M |
| Speichereinheit | MEM |
| Modus | M1, M2, M3 |
| Narkosegasmischeinheit | NG |
| Druck | P |
| Patient | PT |
| Mindestdruck | PEEP |
| Maximaldruckwert, Solldruckwert | Pinsp |
| Maximaldruck | Pmax |
| Ventil | PV |
| Recheneinheit | R |
| Beatmungsfrequenz | RR, f |
| Beatmungsfrequenz | RRmin, f_min |
| Rückschlagventile | RV |
| Schritt | S1, S2, S3, S4, S5, S6 |
| Teilverfahrensschritt | S41, ... , S46, S51, ... , S56, S61, ... , S68, S30, ...,S34 |
| Counter | SB |
| Spalte | SP1, SP2, SP3 |
| Zeitlicher Abstand | T |
| Tabelle | T1, T2, T3, T4, T10, T11, T20 |
| Zeitdauer | Ti |
| Zeitfenster | Tmax |
| unterer Schwellenwert | USW |

(fortgesetzt)

| | |
|---|---|
| Volumenstrom | $\dot{V}$ |
| Vapor | V |
| oberer Volumengrenzwert | VTO1, VTO2 |
| unterer Volumengrenzwert | VTU1, VTU2 |
| Ventilationsgrade | V1, ... , V5 |
| Volumenstromsensor | VS |
| Volumenstromsensorsignal | VSS |
| Tidalvolumen | VT |
| Warnsignal | WS |
| Y-Stück | YS |
| Differenzdruck, Solldruckwert | $\Delta P$ |
| Triggerzeitpunkt | ZP |
| Steuersignal | NGAS |
| Statussignal | SI |
| Speichereinheit | M |
| Warnsignalausgabeeinheit | WSE |
| Betriebsmodus | MO1, MO2, MO3 |
| Druckdifferenz | Pdiff |
| Zieltidalvolumen | TVT |
| Druckanpassungswert | DEP |
| Eingabesignal | E, E2 |
| Ausführungsform des zweiten Betriebsmodus | M1, M2, M3 |
| mittlere alveoläre Anästhesiegaskonzentration | MAC, xMAC |
| Anästhesiegaskonzentrationssignal | AGS |
| Messwerte | MW |
| Zeitpunkt | tX, t1 |
| oberen Grenzwertes | OG |
| unteren Grenzwertes | UG |

**Patentansprüche**

1. Anästhesiebeatmungsvorrichtung (BV) zur automatisierten Beatmung eines Patienten (PT), aufweisend

   - einen exspiratorischen Port (EP) und einen inspiratorischen Port (IP) zum Anschluss eines dem Patienten (PT) zugewandten Beatmungsschlauches (BS) für ein Atemgas,
   - eine Atemgasfördereinheit (AGF),
   - wenigstens einen Volumenstromsensor (VS) zum Erfassen eines Volumenstroms (V) des Atemgases, wenigstens einen Atemgassensor (AS) zum Erfassen einer Kohlendioxidkonzentration sowie wenigstens einen Drucksensor (DS) zum Erfassen eines Drucks (P) des Atemgases
   - und ferner wenigstens eine Recheneinheit (R), welche dazu ausgebildet ist, in einem ersten Betriebsmodus (MO1) die Atemgasfördereinheit (AGF) in Abhängigkeit einer vorgegebenen Beatmungsfrequenz (RR), des erfassten Drucks (P) und eines vorgegebenen Solldruckwertes (Pinsp) anzusteuern,

wobei die Recheneinheit (R) ferner dazu ausgebildet ist,

- in dem ersten Betriebsmodus (MO1) auf Basis des erfassten Volumenstroms und der erfassten Kohlendioxidkonzentration ein Vorliegen eines erwünschten Betriebszustand hinsichtlich der automatisierten Beatmung zu detektieren
- und bei Detektion des Betriebszustandes einen Wechsel in einen zweiten Betriebsmodus (M02) zu ermöglichen, in welchem die Recheneinheit die Atemgasfördereinheit derart ansteuert, dass eine druckgesteuerte oder eine druckunterstützende Beatmung durchgeführt wird,

**dadurch gekennzeichnet, dass** die Recheneinheit (R) dazu ausgebildet ist, in dem ersten Betriebsmodus (MO1)

- auf Basis des erfassten Volumenstroms (V) ein dem Patienten (PT) zugeführtes Tidalvolumen (VT) zu bestimmen ,
- und ferner auf Basis der erfassten Kohlendioxidkonzentration eine endexspiratorische Kohlendioxidkonzentration (etCO2) zu bestimmen sowie ferner eine Anpassung des Solldruckwertes (Pinsp) und eine Anpassung der Beatmungsfrequenz (RR) in Abhängigkeit
- des bestimmten Tidalvolumens (VT),
- eines oberen Volumengrenzwertes (VTO1)
- eines unteren Volumengrenzwertes (VTU1),
- der bestimmten endexspiratorischen Kohlendioxidkonzentration (etCO2),
- eines oberen Konzentrationsgrenzwertes (etCO2O1)
- und eines unteren Konzentrationsgrenzwertes (etCO2U1) vorzunehmen.

2. Anästhesiebeatmungsvorrichtung (BV) nach Anspruch 1,
wobei die Recheneinheit (R) dazu ausgebildet ist, den Wechsel in den zweiten Betriebsmodus (MO2) dann zu ermöglichen, wenn der Betriebszustand innerhalb eines vorgegebenen Zeitraumes detektiert wird.

3. Anästhesiebeatmungsvorrichtung (BV) nach Anspruch 2,
wobei die Recheneinheit dazu ausgebildet ist, den Wechsel in den zweiten Betriebsmodus (MO2) dann automatisch vorzunehmen, wenn der Betriebszustand innerhalb des vorgegebenen Zeitraumes detektiert wird.

4. Anästhesiebeatmungsvorrichtung (BV) nach Anspruch 1 oder 2, wobei die Recheneinheit dazu ausgebildet ist, bei Detektion des Betriebszustandes

- ein Ausgabesignal (AS) auszugeben, welches das Vorliegen des erwünschten Betriebszustandes indiziert,
- sowie in Abhängigkeit eines Eingabesignals (E) in den zweiten Betriebsmodus (MO2) zu wechseln.

5. Anästhesiebeatmungsvorrichtung (BV) nach Anspruch 1,
aufweisend ferner wenigstens einen Atemgassensor (AS) zum Erfassen einer Anästhesiegaskonzentration des Atemgases,
wobei der erwünschte Betriebszustand ein erster erwünschter Betriebszustand ist,
**dadurch gekennzeichnet**, das die Recheneinheit ferner dazu ausgebildet ist, in dem zweiten Betriebsmodus (MO2)

- auf Basis der erfassten Anästhesiegaskonzentration ein Vorliegen eines zweiten erwünschten Betriebszustand hinsichtlich der automatisierten Beatmung zu detektieren
- und bei Detektion des zweiten Betriebszustandes ein zweites Ausgabesignal auszugeben, welches das Vorliegen des zweiten erwünschten Betriebszustandes indiziert.

6. Anästhesiebeatmungsvorrichtung (BV) nach Anspruch 5,
**dadurch gekennzeichnet, dass** die Recheneinheit ferner dazu ausgebildet ist, in dem zweiten Betriebsmodus (MO2) das Vorliegen des zweiten erwünschten Betriebszustand ferner in Abhängigkeit eines Informationssignals (SI), welches einen Betriebszustand eines Vapors (V) indiziert, zu detektieren.

7. Anästhesiebeatmungsvorrichtung (BV) nach Anspruch 5,
**dadurch gekennzeichnet, dass** die Recheneinheit dazu ausgebildet ist, bei Detektion des zweiten Betriebszustandes in Abhängigkeit eines weiteren Eingabesignals (E2) oder aber automatisch in einen dritten Betriebsmodus (MO3) zu wechseln, in welchem die Recheneinheit die Atemgasfördereinheit derart ansteuert, dass eine druckunterstützende Beatmung durchgeführt wird.

8. Anästhesiebeatmungsvorrichtung (BV) nach Anspruch 5,
**dadurch gekennzeichnet, dass** die Recheneinheit (R) dazu ausgebildet ist,
in dem zweiten Betriebsmodus die Atemgasfördereinheit (AGF)

- in Abhängigkeit des erfassten Drucks (P) und eines zweiten vorgegebenen Solldruckwertes (Pinsp, ΔP) für eine druckgesteuerte oder eine druckunterstützende Beatmung anzusteuern,
- auf Basis des erfassten Volumenstroms (V) ein dem Patienten (PT) zugeführtes Tidalvolumen (VT) zu bestimmen ,
- auf Basis der erfassten Kohlendioxidkonzentration eine endexspiratorische Kohlendioxidkonzentration (etCO2) zu bestimmen

sowie ferner eine Anpassung des Solldruckwertes (Pinsp, ΔP) und eine Anpassung einer Beatmungsfrequenz (RR, RRmin) in Abhängigkeit

- des bestimmten Tidalvolumens (VT),
- eines oberen Volumengrenzwertes (VTO1)
- eines unteren Volumengrenzwertes (VTU1),
- der bestimmten endexspiratorischen Kohlendioxidkonzentration (etCO2),
- eines oberen Konzentrationsgrenzwertes (etCO2O1)
- und eines unteren Konzentrationsgrenzwertes (etCO2U1) vorzunehmen.

9. Anästhesiebeatmungsvorrichtung (BV) nach Anspruch 7,
wobei die Recheneinheit (R) dazu ausgebildet ist, in dem dritten Betriebsmodus (MO3) die Atemgasfördereinheit (AGF) in Abhängigkeit des erfassten Drucks (P) und eines vorgegebenen Solldruckwertes (ΔP) anzusteuern,
**dadurch gekennzeichnet, dass** die Recheneinheit (R) ferner dazu ausgebildet ist, in dem dritten Betriebsmodus eine Anpassung des Solldruckwertes (ΔP) in Abhängigkeit der erfassten Anästhesiegaskonzentration vorzunehmen.

10. Recheneinheit (R) für eine Anästhesiebeatmungsvorrichtung (BV) zur automatisierten Beatmung,
ausgebildet zum

- Erfassen eines Volumenstromsignals, welches einen Volumenstrom eines Atemgases indiziert,
- Erfassen eines Kohlendioxidkonzentrationssignals, welches eine Kohlendioxidkonzentration des Atemgases indiziert,
- Erfassen eines Drucksignals, welches einen Druck des Atemgases indiziert,
- in einem ersten Betriebsmodus (MO1), Bereitstellen eines Ansteuersignals ANS für eine Atemgasfördereinheit (AGF), wobei die Recheneinheit (R) das Ansteuersignal (ANS) in Abhängigkeit einer vorgegebenen Beatmungsfrequenz, des erfassten Drucksignals (DSS) und eines vorgegebenen Solldruckwertes (ΔP) bestimmt,

wobei die Recheneinheit (R) ausgebildet ist,

- in dem ersten Betriebsmodus (MO1) auf Basis des erfassten Volumenstroms und der erfassten Kohlendioxidkonzentration ein Vorliegen eines erwünschten Betriebszustand hinsichtlich der automatisierten Beatmung zu detektieren
- und, bei Detektion des Betriebszustandes, einen Wechsel in einen zweiten Betriebsmodus (MO2) zu ermöglichen, in welchem die Recheneinheit die Atemgasfördereinheit derart ansteuert, dass eine druckgesteuerte oder eine druckunterstützende Beatmung durchgeführt wird

**dadurch gekennzeichnet, dass** die Recheneinheit (R) im ersten Betriebsmodus (MO1) ferner ausgebildet ist zum

- Bestimmen eines dem Patienten (PT) zugeführten Tidalvolumens (VT) auf Basis des erfassten Volumen-stromsignals (V),
- Bestimmen einer endexspiratorischen Kohlendioxidkonzentration (etCO2) auf Basis des erfassten Kohlendioxidkonzentrationssignals sowie ferner Anpassen des Solldruckwertes (Pinsp) und Anpassen der Beatmungsfrequenz (RR) in Abhängigkeit
- des bestimmten Tidalvolumens (VT),
- eines oberen Volumengrenzwertes (VTO1)
- eines unteren Volumengrenzwertes (VTU1),
- der bestimmten endexspiratorischen Kohlendioxidkonzentration (etCO2),

- eines oberen Konzentrationsgrenzwertes (etCO2O1) und eines unteren Konzentrationsgrenzwertes (etCO2U1).

**Claims**

1. Anaesthesia ventilator (BV) for the automated ventilation of a patient (PT), having

   - an expiratory port (EP) and an inspiratory port (IP) for the attachment of a ventilation tube (BS) directed towards the patient (PT) and supplying a breathing gas,
   - a breathing gas delivery unit (AGF),
   - at least one volume flow sensor (VS) for detecting a volume flow (V) of the breathing gas, at least one breathing gas sensor (AS) for detecting a carbon dioxide concentration, and at least one pressure sensor (DS) for detecting a pressure (P) of the breathing gas,
   - and moreover at least one computer (R), which is configured, in a first mode of operation (MO1), to actuate the breathing gas delivery unit (AGF) as a function of a pre-set ventilation rate (RR), the detected pressure (P) and a pre-set desired pressure value (Pinsp),

   wherein the computer (R) is moreover configured

   - to detect, in the first mode of operation (MO1), the presence of a desired operating state concerning the automated ventilation, on the basis of the detected volume flow and of the detected carbon dioxide concentration, and,
   - upon detection of the operating state, to permit a change-over to a second mode of operation (MO2) in which the computer actuates the breathing gas delivery unit in such a way that a pressure control ventilation or a pressure support ventilation is carried out,

   **characterized in that** the computer (R) is configured, in the first mode of operation (MO1),

   - to determine, on the basis of the detected volume flow (V), a tidal volume (VT) delivered to the patient (PT),
   - and moreover to determine an end-expiratory carbon dioxide concentration (etCO2) on the basis of the detected carbon dioxide concentration, and moreover to effect an adaptation of the desired pressure value (Pinsp) and an adaptation of the ventilation rate (RR) as a function of
   - the determined tidal volume (VT),
   - an upper volume limit value (VTO1),
   - a lower volume limit value (VTU1),
   - the determined end-expiratory carbon dioxide concentration (etCO2),
   - an upper concentration limit value (etCO2O1),
   - and a lower concentration limit value (etCO2U1).

2. Anaesthesia ventilator (BV) according to Claim 1, wherein the computer (R) is configured to permit the change-over to the second mode of operation (MO2) when the operating state is detected within a pre-set time interval.

3. Anaesthesia ventilator (BV) according to Claim 2, wherein the computer is configured to effect the change-over to the second mode of operation (MO2) automatically when the operating state is detected within the pre-set time interval.

4. Anaesthesia ventilator (BV) according to Claim 1 or 2, wherein the computer, upon detection of the operating state, is configured

   - to output an output signal (AS), which indicates the presence of the desired operating state,
   - and to change over to the second mode of operation (MO2) as a function of an input signal (E).

5. Anaesthesia ventilator (BV) according to Claim 1, moreover having at least one breathing gas sensor (AS) for detecting an anaesthetic gas concentration of the breathing gas, wherein the desired operating state is a first desired operating state, **characterized in that** the computer is moreover configured, in the second operating mode (MO2),

- to detect the presence of a second desired operating state concerning the automated ventilation, on the basis of the detected anaesthetic gas concentration,
- and, upon detection of the second operating state, to output a second output signal, which indicates the presence of the second desired operating state.

6. Anaesthesia ventilator (BV) according to Claim 5, **characterized in that** the computer is moreover configured, in the second mode of operation (MO2), to detect the presence of the second desired operating state also as a function of an information signal (SI), which indicates an operating state of a vapour (V).

7. Anaesthesia ventilator (BV) according to Claim 5, **characterized in that** the computer is configured, upon detection of the second operating state, to change over to a third mode of operation (MO3) as a function of a further input signal (E2) or else automatically, in which third mode of operation (MO3) the computer actuates the breathing gas delivery unit in such a way that a pressure support ventilation is performed.

8. Anaesthesia ventilator (BV) according to Claim 5, **characterized in that** the computer (R) is configured, in the second mode of operation (MO2),

- to actuate the breathing gas delivery unit (AGF) for a pressure control ventilation or a pressure support ventilation, as a function of the detected pressure (P) and of a second pre-set desired pressure value (Pinsp, $\Delta$P),
- to determine, on the basis of the detected volume flow (V), a tidal volume (VT) delivered to the patient (PT),
- to determine an end-expiratory carbon dioxide concentration (etCO2) on the basis of the detected carbon dioxide concentration

and moreover to effect an adaptation of the desired pressure value (Pinsp, $\Delta$P) and an adaptation of a ventilation rate (RR, RRmin) as a function of

- the determined tidal volume (VT),
- an upper volume limit value (VTO1),
- a lower volume limit value (VTU1),
- the determined end-expiratory carbon dioxide concentration (etCO2),
- an upper concentration limit value (etCO2O1), and
- a lower concentration limit value (etCO2U1).

9. Anaesthesia ventilator (BV) according to Claim 7,
wherein the computer (R) is configured, in the third mode of operation (MO3), to actuate the breathing gas delivery unit (AGF) as a function of the detected pressure (P) and of a pre-set desired pressure value ($\Delta$P),
**characterized in that** the computer (R) is moreover configured, in the third mode of operation, to effect an adaptation of the desired pressure value ($\Delta$P) as a function of the detected anaesthetic gas concentration.

10. Computer (R) for an anaesthesia ventilator (BV) for automated ventilation, configured

- to detect a volume flow signal, which indicates a volume flow of a breathing gas,
- to detect a carbon dioxide concentration signal, which indicates a carbon dioxide concentration of the breathing gas,
- to detect a pressure signal, which indicates a pressure of the breathing gas,
- to provide, in a first mode of operation (MO1), an actuation signal ANS for a breathing gas delivery unit (AGF), wherein the computer (R) determines the actuation signal (ANS) as a function of a pre-set ventilation rate, of the detected pressure signal (DSS) and of a pre-set desired pressure value ($\Delta$P),

wherein the computer (R) is configured

- to detect, in the first mode of operation (MO1), the presence of a desired operating state concerning the automated ventilation, on the basis of the detected volume flow and of the detected carbon dioxide concentration,
- and, upon detection of the operating state, to permit a change-over to a second mode of operation (MO2), in which the computer actuates the breathing gas delivery unit in such a way that a pressure control ventilation or a pressure support ventilation is performed,

**characterized in that** the computer (R), in the first mode of operation (MO1), is moreover configured

- to determine, on the basis of the detected volume flow signal (V), a tidal volume (VT) delivered to the patient (PT),
- to determine an end-expiratory carbon dioxide concentration (etCO2) on the basis of the detected carbon dioxide concentration signal and moreover to adapt the desired pressure value (Pinsp) and adapt the ventilation rate (RR) as a function of
  - the determined tidal volume (VT),
  - an upper volume limit value (VTO1),
  - a lower volume limit value (VTU1),
  - the determined end-expiratory carbon dioxide concentration (etCO2),
  - an upper concentration limit value (etCO2O1), and
  - a lower concentration limit value (etCO2U1).

**Revendications**

1. Dispositif de ventilation pour anesthésie (BV), pour la ventilation automatisée d'un patient (PT), présentant :

   - un port expiratoire (EP) et un port inspiratoire (IP) pour le raccordement d'un tuyau de ventilation (BS) tourné vers le patient (PT) pour un gaz respiratoire,
   - une unité de transport de gaz respiratoire (AGF),
   - au moins un détecteur de débit volumique (VS) pour détecter un débit volumique (V) du gaz respiratoire, au moins un capteur de gaz respiratoire (AS) pour détecter une concentration en dioxyde de carbone ainsi qu'au moins un capteur de pression (DS) pour détecter une pression (P) du gaz respiratoire,
   - et en outre au moins une unité de calcul (R) qui est réalisée pour piloter, dans un premier mode de fonctionnement (MO1), l'unité de transport de gaz respiratoire (AGF) en fonction d'une fréquence de ventilation prédéfinie (RR), de la pression détectée (P) et d'une valeur de pression de consigne prédéfinie (Pinsp), l'unité de calcul (R) étant en outre réalisée,
   - dans le premier mode de fonctionnement (MO1), sur la base du débit volumique détecté et de la concentration en dioxyde de carbone détectée, pour détecter la présence d'un état de fonctionnement souhaité en rapport avec la respiration automatisée,
   - et dans le cas de la détection d'un état de fonctionnement, pour permettre un passage à un deuxième mode de fonctionnement (MO2) dans lequel l'unité de calcul pilote l'unité de transport de gaz respiratoire de telle sorte qu'une ventilation commandée en pression ou à support en pression soit effectuée,

   **caractérisé en ce que** l'unité de calcul (R), dans le premier mode de fonctionnement (MO1), est réalisée

   - pour déterminer, sur la base du débit volumique détecté (V), un volume courant (VT) acheminé au patient (PT),
   - et de plus, sur la base de la concentration en dioxyde de carbone détecté, pour déterminer une concentration en dioxyde de carbone expiratoire finale (etCO2) ainsi que pour effectuer une adaptation de la valeur de pression de consigne (Pinsp) et une adaptation de la fréquence de ventilation (RR) en fonction
     - du volume courant déterminé (DT),
     - d'une valeur limite de volume supérieure (VTO1),
     - d'une valeur limite de volume inférieure (VTU1),
     - de la concentration en dioxyde de carbone expiratoire finale déterminée (etCO2),
     - d'une valeur limite de concentration supérieure (etCO2O1),
     - et d'une valeur limite de concentration inférieure (etCO2U1).

2. Dispositif de ventilation pour anesthésie (BV) selon la revendication 1,
   dans lequel l'unité de calcul (R) est réalisée pour permettre le passage au deuxième mode de fonctionnement (MO2) lorsque l'état de fonctionnement est détecté à l'intérieur d'un intervalle de temps prédéfini.

3. Dispositif de ventilation pour anesthésie (BV) selon la revendication 2,
   dans lequel l'unité de calcul est réalisée pour effectuer automatiquement le passage au deuxième mode de fonctionnement (MO2) lorsque l'état de fonctionnement est détecté à l'intérieur de l'intervalle de temps prédéfini.

4. Dispositif de ventilation pour anesthésie (BV) selon la revendication 1 ou 2,
   dans lequel l'unité de calcul est réalisée, à la détection de l'état de fonctionnement,

   - pour émettre un signal de sortie (AS) qui indique la présence de l'état de fonctionnement souhaité,

- et, en fonction d'un signal d'entrée (E), pour passer au deuxième mode de fonctionnement (MO2).

**5.** Dispositif de ventilation pour anesthésie (BV) selon la revendication 1,
présentant en outre au moins un détecteur de gaz respiratoire (AS) pour détecter une concentration en gaz d'anesthésie du gaz respiratoire,
l'état de fonctionnement souhaité étant un premier état de fonctionnement souhaité,
**caractérisé en ce que** l'unité de calcul est en outre réalisée, dans le deuxième mode de fonctionnement (MO2),

- pour détecter, sur la base de la concentration en gaz d'anesthésie détectée, une présence d'un deuxième état de fonctionnement souhaité relatif à la ventilation automatisée,
- et, lors de la détection du deuxième état de fonctionnement, pour émettre un deuxième signal de sortie qui indique la présence du deuxième état de fonctionnement souhaité.

**6.** Dispositif de ventilation pour anesthésie (BV) selon la revendication 5,
**caractérisé en ce que** l'unité de calcul est en outre réalisée, dans le deuxième mode de fonctionnement (MO2),
pour détecter la présence du deuxième état de fonctionnement souhaité en outre en fonction d'un signal d'information (SI) qui indique un état de fonctionnement d'une vapeur (V).

**7.** Dispositif de ventilation pour anesthésie (BV) selon la revendication 5,
**caractérisé en ce que** l'unité de calcul est réalisée, lors de la détection du deuxième état de fonctionnement, en fonction d'un signal d'entrée supplémentaire (E2) ou bien automatiquement, pour passer à un troisième mode de fonctionnement (MO3) dans lequel l'unité de calcul pilote l'unité de transport de gaz respiratoire de telle sorte qu'une ventilation à support en pression soit effectuée.

**8.** Dispositif de ventilation pour anesthésie (BV) selon la revendication 5,
**caractérisé en ce que** l'unité de calcul (R) est réalisée,
dans le deuxième mode de fonctionnement,

- pour piloter l'unité de transport de gaz respiratoire (AGF) en fonction de la pression détectée (P) et d'une deuxième valeur de pression de consigne prédéfinie (Pinsp, ∆P) pour une ventilation commandée en pression ou à support en pression,
- sur la base du débit volumique détecté (V), pour déterminer un volume courant (VT) acheminé au patient (PT),
- sur la base de la concentration en dioxyde de carbone détecté, pour déterminer une concentration dioxyde de carbone expiratoire final (etCO2),

et en outre pour effectuer une adaptation de la valeur de pression de consigne (Pinsp, ∆P) et une adaptation d'une fréquence de ventilation (RR, RRmin) en fonction

- du volume courant déterminé (VT),
- d'une valeur limite de volume supérieure (VTO1),
- d'une valeur limite de volume inférieure (VTU1),
- de la concentration en dioxyde de carbone expiratoire finale déterminée (etCO2),
- d'une valeur limite de concentration supérieure (etCO2O1),
- et d'une valeur limite de concentration inférieure (etCO2U1).

**9.** Dispositif de ventilation pour anesthésie (BV) selon la revendication 7,
dans lequel l'unité de calcul (R) est réalisée, dans le troisième mode de fonctionnement (MO3), pour piloter l'unité de transport de gaz respiratoire (AGF) en fonction de la pression détectée (P) et d'une valeur de pression de consigne prédéfinie (∆P),
**caractérisé en ce que** l'unité de calcul (R) est en outre réalisé, dans le troisième mode de fonctionnement, pour effectuer une adaptation de la valeur de pression de consigne (∆P) en fonction de la concentration en gaz d'anesthésie détectée.

**10.** Unité de calcul (R) pour un dispositif de ventilation pour anesthésie (BV) pour une ventilation automatisée,
réalisée pour

- détecter un signal de débit volumique qui indique un débit volumique d'un gaz respiratoire,
- détecter un signal de concentration en dioxyde de carbone qui indique une concentration en dioxyde de carbone du gaz respiratoire,

- détecter un signal de pression qui indique une pression du gaz respiratoire,
- dans un premier mode de fonctionnement (MO1), fournir un signal de commande (ANS) pour une unité de transport de gaz respiratoire (AGF), l'unité de calcul (R) déterminant le signal de commande (ANS) en fonction d'une fréquence de ventilation prédéfinie, du signal de pression détecté (DSS) et d'une valeur de pression de consigne prédéfinie ($\Delta$P),

l'unité de calcul (R) étant réalisée

- dans le premier mode de fonctionnement (MO1), sur la base du débit volumique détecté et de la concentration en dioxyde de carbone détecté, pour détecter la présence d'un état de fonctionnement souhaité en rapport avec la ventilation automatisée,
- et, lors de la détection de l'état de fonctionnement, pour permettre un passage à un deuxième mode de fonctionnement (MO2) dans lequel l'unité de calcul pilote l'unité de transport de gaz respiratoire de telle sorte qu'une ventilation commandée en pression ou à support en pression soit effectuée,

**caractérisée en ce que** l'unité de calcul (R), dans le premier mode de fonctionnement (MO1), est en outre réalisée pour

- déterminer un volume courant (VT) acheminé au patient (PT) sur la base du signal de débit volumique détecté (V),
- déterminer une concentration en dioxyde de carbone expiratoire finale (etCO2) sur la base du signal de concentration en dioxyde de carbone détecté

et en outre pour adapter la valeur de pression de consigne (Pinsp) et adapter la fréquence de ventilation (RR) en fonction

- du volume courant déterminé (VT),
- d'une valeur limite de volume supérieure (VTO1),
- d'une valeur limite de volume inférieure (VTU1),
- de la concentration en dioxyde de carbone expiratoire finale déterminée (etCO2),
- d'une valeur limite de concentration supérieure (etCO2O1),
- et d'une valeur limite de concentration inférieure (etCO2U1).

**FIG. 1**

FIG. 2

FIG. 3

Pinsp

ΔP

PEEP

Ti

$\frac{1}{f} \triangleq T$

Triggerfenster zum
insp. Synchronisieren

f = RR

ohne
Spontanatmung

mit
Spontanatmung

FIG. 4

EP 3 383 466 B1

FIG. 5

**FIG. 6**

```
                          ┌──────────┐
                          │  Input   │──── S1
                          └────┬─────┘
                               │
                               ▼
                  ┌──────────────────────┐
                  │  RR: = 12/min        │
                  │  Pinsp: = 18 mbar    │──── S2
                  │  PEEP: = 5 mbar      │
                  └──────────┬───────────┘
                             │
                             ▼
                      ┌─────────────┐
                      │  set KOZ    │──── S3
                      └──────┬──────┘
                             │
                        ⧗    ▼
                      ┌─────────────┐              ┌──── S30
           ┌────────▶ │   15 sec    │ ◀──────────────────────────────────┐
           │          └──────┬──────┘     S4                              │
           │      S5         │                                           │
           │                 ▼         > 5min    ╭─────────╮              │
           │            ◇ duration ◇ ──────────▶│  abort  │              │
           │                 │                   ╰─────────╯              │
           │              ≤ 5min                                         │
           │                 ▼                                           │
           │          ┌─────────────┐                                    │
           │          │ determine   │──── S6                             │
           │          │ VT, etCO2   │                                    │
           │          └──────┬──────┘                                    │
           │                 ▼                                           │
           │          ┌─────────────┐  S7                               │
           │          │    CoV      │                                    │
           │          └──────┬──────┘                                    │
           │    S9           │         S8                               │
           │   ┌──────────┐  ▼ not o.k.                                 │
           │   │determine │◀─── ◇ VT? ◇                                 │
           │   │  Pdiff   │                                             │
           │   └────┬─────┘     │ o.k.   S11                            │
           │        ▼           ▼                                       │
           │  ┌──────────────┐  ◇ etCO2? ◇ ──── not o.k.                │
           │  │ Pinsp: =     │     │         severe  mild  severe  mild │
           └──│ Pinsp + Pdiff│     │ o.k.    hypov.  hypov. hyperv. hyperv.
              └──────────────┘     ▼          ┌────────┐                 │
                   S10          ⬠ "status     │RR+ = 2 │  ┌────────┐     │
                S16 ─           o.k." ⬠       └───┬────┘  │RR+ = 1 │     │
                                   │               │      └───┬────┘     │
                     S17           ▼               │          │  ┌────────┐
                              ┌─────────┐    E      │          │  │RR- = 2 │
                              │  Input  │◀────      │          │  └───┬────┘
                              └────┬────┘           │          │      │ ┌────────┐
                                   │                │          │      │ │RR- = 1 │
                                   │                │          │      │ └───┬────┘
            ┈┈┈┈┈┈┈┈┈┈┈┈┈┈┈┈┈┈┈┈┈┈│┈┈┈┈┈┈┈┈┈┈┈┈┈┈┈ │┈┈┈┈┈┈┈┈┈┈│┈┈┈┈┈┈│
   ┌────┐              ◇ Mode? ◇       ┌────┐        └──────────┴──────┘
   │ M1 │◀───────────            ─────▶│ M3 │            MO1
   └────┘                 │            └────┘
     S21                  ▼  S18          S23
                     ┌────────┐
           S22 ──    │   M2   │                        MO2
                     └────────┘
```

FIG. 7

# FIG. 8

**FIG. 9**

T1

| Input | Valid Range | |
|-------|-------------|---|
| Level of ventilation | normal ventilated, mild hyperventilated | ⌐ SP1 |
| Lung mechanic | normal, restrictive, obstructive | ⌐ SP2 |

**FIG. 10**

T2

| Level of ventilation | | | |
|---|---|---|---|
| normal ventilated | Lung mechanic | | |
| KOZ Parameter | normal | obstructive | restrictive |
| etCO$_2$U1 [mmHg] | 35 | 45 | 35 |
| etCO$_2$O1 [mmHg] | 45 | 45 | 45 |
| Level of ventilation | | | |
| mild hyperventilated | Lung mechanic | | |
| KOZ Parameter | normal | obstructive | restrictive |
| etCO$_2$U1 [mmHg] | 30 | 40 | 30 |
| etCO$_2$O1 [mmHg] | 40 | 50 | 40 |
| | Lung mechanic | | |
| KOZ Parameter | normal | obstructive | restrictive |
| VTU1 [ml/kg] | 7 | 3 | 2 |
| VTO1 [ml/kg] | 10 | 10 | 6 |

**FIG. 11**

T3

| Classification of Ventilation ($V_T$) (CoV_VT) | |
|---|---|
| $V_T$ | $V_T$-Range |
| very low | VT < VTU2 |
| low | VTU2 < VT < VTU1 |
| normal | VTU1 ≤ VT ≤ VTO1 |
| high | VTO2 > VT > VTO1 |
| very high | VT > VTO2 |

T4

| Classification of Ventilation $etCO_2$ (CoV_$etCO_2$) | |
|---|---|
| $etCO_2$ | $etCO_2$.Range |
| severe hyperventilated | $etCO_2$ < $etCO_2$U2 |
| mild hyperventilated | $etCO_2$U2 < $etCO_2$ < $etCO_2$U1 |
| normoventilated | $etCO_2$U1 ≤ $etCO_2$ ≤ $etCO_2$O1 |
| mild hypoventilated | $etCO_2$O2 > $etCO_2$ > $etCO_2$O1 |
| severe hypoventilated | $etCO_2$ > $etCO_2$O2 |

## FIG. 12

**FIG. 13**

EP 3 383 466 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

*   DE 102013002408

**In der Beschreibung aufgeführte Nicht-Patentliteratur**

*   **ERFINDER STEFAN MERSMANN ; WILFRIED BUSCHKE ; PROF. CHRISTOPH HÖRMANN.** Beatmungsvorrichtung und Verfahren zur automatisierten Beatmung eines Patienten. Anmelderin Drägerwerk AG & Co. KGaA, 02. Dezember 2015
*   **KARIN DEDEN.** Beatmungsmodi in der Intensivmedizin. Dräger Medical GmbH
*   Gebrauchsanweisung"Zeus Infinity Empowered. Dräger Medical AG & Co. KG, Februar 2009
*   Primus Infinity Empowered. Dräger Medical GmbH, 03. September 2010, 132-134
*   **ERFINDER STEFAN MERSMANN ; WILFRIED BUSCHKE ; PROF. CHRISTOPH HÖRMANN.** Anästhesiebeatmungsvorrichtung zur automatisierten Beatmung eines Patienten. Anmelderin Drägerwerk AG & Co. KGaA, 02. Dezember 2015